# EUROPEAN PATENT SPECIFICATION

(11) **EP 3 585 479 B1**
(45) Date of publication and mention of the grant of the patent: **18.12.2024**
(21) Application number: 18710612.5
(22) Date of filing: 26.02.2018
(51) Int. Cl.: A61N 1/39, A61N 1/04, A61B 5/00, A61N 1/32

(54) **SUPPORT GARMENT FOR A WEARABLE MEDICAL DEVICE**
STÜTZBEKLEIDUNG FÜR EINE MEDIZINISCHE WEARABLE-VORRICHTUNG
VÊTEMENT DE SUPPORT POUR DISPOSITIF MÉDICAL METTABLE

(30) Priority: 27.02.2017 US 201715443856
(43) Date of publication of application: 01.01.2020
(73) Proprietor: Zoll Medical Corporation, Chelmsford, MA 01824-4105 (US)
(72) Inventor: HILL, Erin, Elaine, Pittsburgh Pennsylvania 15239 (US); PAVEL, Trisha, A., Pittsburgh Pennsylvania 15223 (US); SWENGLISH, Christopher, Lawrence, Connellsville Pennsylvania 15425 (US); OWENS, Mark, Jerome, Wexford Pennsylvania 15090 (US); SKALOS, Phil, Pittsburgh Pennsylvania 15227 (US)
(74) Representative: Potter Clarkson
(86) International application number: PCT/US2018/019618
(87) International publication number: WO 2018/157017

(56) References cited:
- WO-A1-2015/056262
- DE-A1- 102005 060 985
- US-A1- 2008 287 769
- US-A1- 2008 287 770
- US-A1- 2012 283 794

## Description

### TECHNICAL FIELD

The present disclosure relates to a support garment for a wearable cardiac monitoring and therapeutic medical device, such as a wearable defibrillator, that houses the device and its associated sensing and therapeutic energy delivery electrodes and also provides support for front upper torsal female anatomy.

### BACKGROUND

One of the most deadly forms of heart arrhythmias is ventricular fibrillation, which occurs when the normal, regular electrical impulses are replaced by irregular and rapid impulses, causing the heart muscle to stop normal contractions and to begin to quiver. Normal blood flow ceases, and organ damage or death can result in minutes if normal heart contractions are not restored. Although frequently not noticeable to the victim, ventricular fibrillation is often preceded by ventricular tachycardia, which is a regular but fast rhythm of the heart. Because the victim has no noticeable warning of the impending fibrillation, death often occurs before the necessary medical assistance can arrive.

Because time delays in applying the corrective electrical treatment can result in death, pacemakers and defibrillators have significantly improved the ability to treat these otherwise life-threatening conditions. Normal heart function often can be restored to a person suffering ventricular fibrillation or ventricular tachycardia by a procedure known as cardioversion, the synchronized application of electrical therapy to the heart muscle. Pacemakers and defibrillators that apply corrective electrical pulses externally to the patient's chest wall also are used to correct such life-threatening arrhythmias, but suffer from a drawback insofar as it cannot be possible to apply the device in time during an acute arrhythmic emergency to save the patient's life. Such treatment is needed within a few minutes to be effective.

Consequently, when a patient is deemed at high risk of death from such arrhythmias, electrical devices often are implanted so as to be readily available when treatment is needed. However, patients that have recently had a heart attack or are awaiting such an implantable device can be kept in a hospital where corrective electrical therapy is generally close at hand. Long-term hospitalization is frequently impractical due to its high cost, or due to the need for patients to engage in normal daily activities.

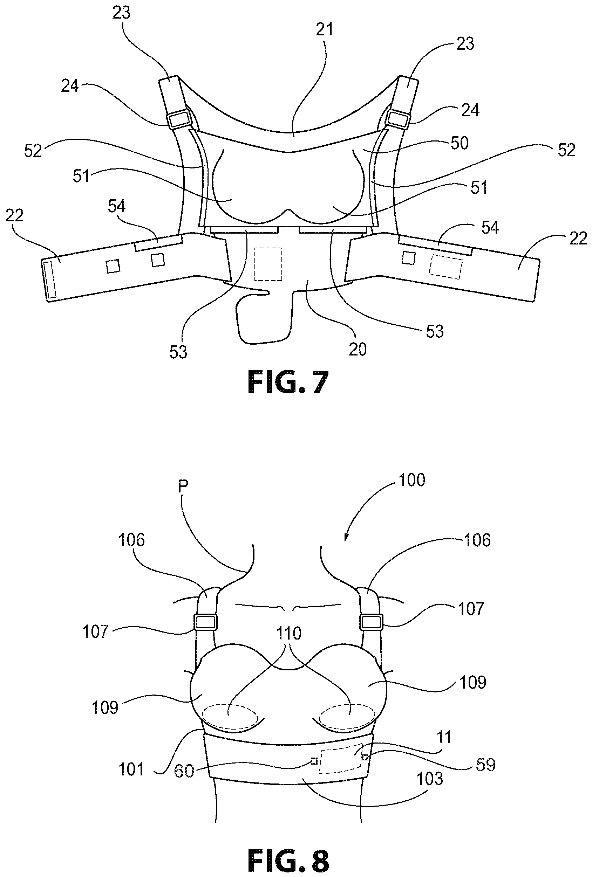

WO2015056262A1 relates to a wearable health monitoring system for use on a daily basis. US2008287769A1 discloses a garment accessory including a member having first and second ends, with the length of the member between the first and second ends being less than the circumference of a subject that the member is configured for and a pair of fastener mechanisms disposed in proximity to the first and second ends of the member, the fastener mechanisms configured to attach the member to an article of clothing worn by a subject. US 2008/0287770 discloses a "garment for ambulatory, physiological monitoring of a patient includes a belt, having first and second end portion with closures at the end portions to wrap around a user's chest, a strap having a first end coupled to a portion of the belt with the strap having a second end, a pair of shoulder strap portions" and "a back portion that joins the second ends of the pair of shoulder strap portions, with at least one of the belt, strap portions and back portion having an accommodation for carrying a sensor." It is also disclosed in US2008/0287770 that "The harness (Y shaped, single strap, and suspenders) can all be worn comfortably with a conventional bra" which is shown in FIG. 8 of the document.

Wearable defibrillators have been developed for patients that have recently experienced cardiac arrest, that are susceptible to heart arrhythmias and are at temporary risk of sudden death, and that are awaiting an implantable device. Support garments have been developed for housing the components of such wearable defibrillators, particularly the sensing and therapeutic energy delivery electrodes, such that the electrodes are properly positioned against the patient's skin. However, such support garments do not provide specific support for the front upper torsal anatomy of female patients.

Accordingly, a need exists for a support garment for a cardiac monitoring and/or treatment device, such as a wearable defibrillator, that incorporates structure configured to support front upper torsal female anatomy.

### SUMMARY

The invention is defined by the claims. Non-limiting examples of the disclosure will now be described.

In an example, a support garment for supporting a patient wearable defibrillator is provided. The support garment is made from a fabric having an outside surface and an inside surface and is configured to be worn about a chest of a patient. The support garment comprises: a back portion; a belt defined by side portions extending from the back portion around a front of the patient; and shoulder straps configured to be attached to the back portion and the belt. The back portion and the belt are configured to support a plurality of sensing electrodes and at least one therapeutic defibrillator electrode on the inside surface thereof. The support garment further comprises at least one bra portion detachably connected to the belt and the shoulder straps, so as to be detachable from the support garment. The at least one bra portion is configured to support front upper torsal female anatomy, the at least one bra portion comprising two cups configured to support the patient's breasts.

The shoulder straps can be adjustable.

The shoulder straps can be configured to be selectively attached to the belt at the front of the patient.

The fabric of the support garment can comprise an elastic, low spring rate material.

The support garment can further comprise at least one pocket configured to receive the at least one therapeutic defibrillator electrode at the front or back of the patient.

The back portion and belt of the support garment can be configured to distribute the plurality of sensing electrodes around a circumference of the chest of the patient.

The bra portion can be connected to the belt by at least one of the following: hook and pile fasteners, a clasp, a button, a snap, a mateable buckle, and a mateable slide connector.

The bra portion can further comprise a halter strap connected to a top of the bra portion proximate to both lateral sides thereof, the halter strap being configured to extend around a neck of the patient.

The bra portion can comprise flaps at both lateral sides thereof, the flaps being configured to wrap around the straps and comprising fastening mechanisms to secure ends of the flaps.

The bra portion, the belt, and the straps can comprise corresponding mateable slide connectors configured to connect the bra band to the belt and the straps.

The at least one bra portion can comprise two bra portions configured to be connected to the shoulder straps, and the belt, each of the two bra portions comprising a cup configured to contact and support one of the patient's breasts.

The two bra portions can comprise a closure mechanism configured to connect the bra portions at the front of the patient.

The belt and the bra portions can comprise a common closure mechanism configured to connect the bra portions and ends of the belt at the front of the patient.

The two bra portions can be disposed between the straps and the belt with each of the two cup portions being connected to a respective belt portion.

Each of the two bra portions can be connected to the respective belt portion and the respective strap by at least one of the following: hook and pile fasteners, a clasp, a button, a snap, a mateable buckle, and a mateable slide connector.

The at least one bra portion can comprise an adjustment mechanism configured to adjust a fit of the bra portion to the patient.

### BRIEF DESCRIPTION OF THE DRAWINGS

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limit of the invention.

Further features and other examples and advantages will become apparent from the following detailed description made with reference to the drawings.
FIG. 1 is a schematic of an exemplary wearable cardiac monitoring and therapeutic medical device that may be used in connection with the present disclosure.
FIG. 2 is a front view of an exemplary support garment and electrode assembly for the wearable cardiac monitoring and therapeutic medical device that may be used in connection with the present disclosure.
FIG. 3 is a rear view of the support garment of FIG. 2.
FIG. 4 is a front view of the support garment of FIG. 2 as worn on a patient.
FIG. 5 is a rear view of the support garment of FIG. 2 as worn on a patient.
FIG. 6 is a front view of a support garment for a wearable cardiac monitoring and therapeutic medical device as worn on a patient in accordance with an example of the present disclosure.
FIG. 7 is a front view of the support garment of FIG. 6.
FIG. 8 is a front view of a support garment for a wearable cardiac monitoring and therapeutic medical device as worn on a patient in accordance with another example of the present disclosure.
FIG. 9 is a rear view of the support garment of FIG. 8 as worn on a patient.
FIG. 10 is a view of an inside surface of a front portion of the support garment of FIG. 8.
FIG. 11 is a view of an inside surface of a rear portion of the support garment of FIG. 8.
FIG. 12 is a front view of a support garment for a wearable cardiac monitoring and therapeutic medical device as worn on a patient in accordance with another example of the present disclosure.
FIG. 13 is a front view of the support garment of FIG. 12.
FIG. 14 is a front view of a support garment for a wearable cardiac monitoring and therapeutic medical device in accordance with another example of the present disclosure.
FIG. 15 is a front view of a support garment for a wearable cardiac monitoring and therapeutic medical device as worn on a patient in accordance with another example of the present disclosure.
FIG. 16 is a front view of the support garment of FIG. 15.
FIG. 17 is a front view of a support garment for a wearable cardiac monitoring and therapeutic medical device in accordance with another example of the present disclosure.
FIG. 18 is a front view of a support garment for a wearable cardiac monitoring and therapeutic medical device in accordance with another example of the present disclosure.
FIG. 19 is a front view of a support garment for a wearable cardiac monitoring and therapeutic medical device in accordance with another example of the present disclosure.
FIG. 20 is a front view of a support garment for a wearable cardiac monitoring and therapeutic medical device in accordance with another example of the present disclosure.
FIG. 21 is a view of a modular support garment for a wearable cardiac monitoring and therapeutic medical device in accordance with another example of the present disclosure.
FIGS. 22A and 22B are top and side views of an exemplary adjustment mechanism for a support garment.
FIG. 23 is a front view of a support garment incorporating another exemplary adjustment mechanism.
FIGS. 24A and 24B are views of an exemplary fastening mechanism for a support garment in the unfastened and fastened states.
FIGS. 25A and 25B are views of another exemplary fastening mechanism for a support garment in the unfastened and fastened states.
FIGS. 26A and 26B are views of another exemplary fastening mechanism for a support garment in the unfastened and fastened states.
FIGS. 27A and 27B are views of another exemplary fastening mechanism for a support garment in the unfastened and fastened states.

### DESCRIPTION OF THE INVENTION

As used herein, the singular form of "a", "an", and "the" include plural referents unless the context clearly dictates otherwise.

As used herein, the terms "right", "left", "top", and derivatives thereof shall relate to the invention as it is oriented in the drawing figures. However, it is to be understood that the invention can assume various alternative orientations and, accordingly, such terms are not to be considered as limiting. Also, it is to be understood that the invention can assume various alternative variations and stage sequences, except where expressly specified to the contrary. It is also to be understood that the specific devices and processes illustrated in the attached drawings, and described in the following specification, are examples. Hence, specific dimensions and other physical characteristics related to the embodiments disclosed herein are not to be considered as limiting.

Notwithstanding that the numerical ranges and parameters setting forth the broad scope of the invention are approximations, the numerical values set forth in the specific examples are reported as precisely as possible. Any numerical value, however, inherently contain certain errors necessarily resulting from the standard deviation found in their respective testing measurements. Also, it should be understood that any numerical range recited herein is intended to include all sub-ranges subsumed therein. For example, a range of "1 to 10" is intended to include any and all sub-ranges between and including the recited minimum value of 1 and the recited maximum value of 10, that is, all subranges beginning with a minimum value equal to or greater than 1 and ending with a maximum value equal to or less than 10, and all subranges in between, e.g., 1 to 6.3, or 5.5 to 10, or 2.7 to 6.1.

As used herein, the terms "communication" and "communicate" refer to the receipt or transfer of one or more signals, messages, commands, or other type of data. For one unit or component to be in communication with another unit or component means that the one unit or component is able to directly or indirectly receive data from and/or transmit data to the other unit or component. This can refer to a direct or indirect connection that can be wired and/or wireless in nature. Additionally, two units or components can be in communication with each other even though the data transmitted can be modified, processed, routed, and the like, between the first and second unit or component. For example, a first unit can be in communication with a second unit even though the first unit passively receives data, and does not actively transmit data to the second unit. As another example, a first unit can be in communication with a second unit if an intermediary unit processes data from one unit and transmits processed data to the second unit. It will be appreciated that numerous other arrangements are possible.

This disclosure relates to systems and techniques for interfacing cardiac monitoring and therapeutic devices with one or more cardiac monitoring and/or therapeutic electrodes attached to a patient. A cardiac monitoring and therapeutic device is configured to monitor a patient for a predetermined cardiac related physiologic condition, e.g., a cardiac arrhythmia, and provide a treatment on detecting the condition. Such a device can include an automated external defibrillator (AED), a wearable cardioverter defibrillator (WCD), or an external cardiac pacing device. The cardiac monitoring and/or therapeutic device as described herein can be ambulatory, e.g., the device is capable of and designed for moving with the patient.

The cardiac device is capable of extended and continuous (e.g., substantially continuous) use by the patient. In some implementations, the continuous use may be substantially continuous in nature. That is, the cardiac device may be continuously used, except for sporadic periods during which the use temporarily ceases (e.g., while the patient bathes, while the patient is refit with a new and/or a different garment, while the battery is charged/changed, while the garment is laundered, etc.). For example, such substantially continuous use as described herein may nonetheless qualify as continuous use.

The cardiac device is also capable of extended and /or long-term use. For example, the cardiac device can be configured to be used by the patient for hours, days, weeks, months, or even years. In some examples, the cardiac device may be continuously used by a patient for a period of at least one week. In some examples, the cardiac device may be continuously used by a patient for a period of at least 30 days. In some examples, the cardiac device may be continuously used by a patient for a period longer at least one month. In some examples, the cardiac device may be continuously used by a patient for a period of at least two months. In some examples, the cardiac device may be continuously used by a patient for a period of at least three months. In some examples, the cardiac device may be continuously used by a patient for a period of at least six months. In some examples, the cardiac device may be continuously used by a patient for a period of at least one year. In some implementations, the extended use may be continuous in nature. The use (e.g., the continuous and/or extended use) of the wearable medical device can include continuous wear by the patient, continuous attachment to the patient, and/or continuous monitoring of the patient. For example, the continuous use can include continuous wear or attachment of the device to the patient, e.g., through one or more of the electrodes as described herein, during both periods of monitoring and periods when the device may not be monitoring the patient but is otherwise still worn by or otherwise attached to the patient. The cardiac device is configured to continuously monitor the patient for cardiacrelated information (e.g., ECG information, including arrhythmia information, heart sounds, etc.) and/or non-cardiac information (e.g., blood oxygen, the patient's temperature, glucose levels, tissue fluid levels, and/or lung sounds). The cardiac device may carry out its monitoring in periodic or aperiodic time intervals or times. For example, the monitoring during intervals or times can be triggered by a user action or another event.

As noted above, the cardiac device can be configured to monitor other physiologic parameters of the patient in addition to cardiac related parameters. For example, the device can be configured to monitor, for example, lung sounds (e.g., using microphones and/or accelerometers), breath sounds, sleep related parameters (e.g., snoring, sleep apnea), tissue fluids (e.g., using radio-frequency transmitters and sensors), among others.

Other example external cardiac monitoring and therapeutic devices capable of interfacing with the electrode connectors disclosed herein include automated cardiac monitors and/or defibrillators for use in certain specialized conditions and/or environments such as in combat zones or within emergency vehicles. Such devices can be configured so that they can be used immediately (or substantially immediately) in a life-saving emergency. In some examples, the defibrillators described herein can be pacing-enabled, e.g., capable of providing therapeutic pacing pulses to the patient.

### Exemplary Monitoring and Treatment Device:

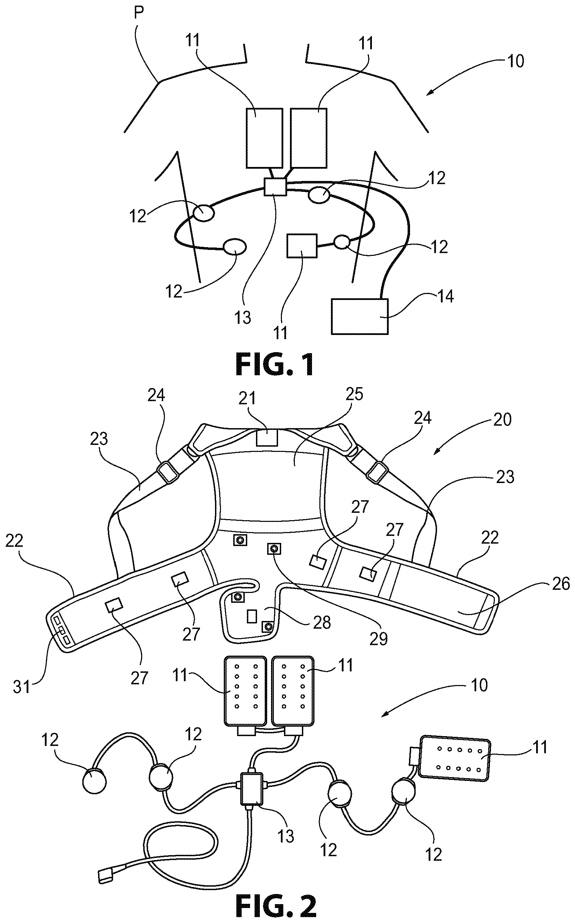

FIG. 1 illustrates an exemplary therapeutic medical device 14 that is external, ambulatory, and wearable by a patient P, and configured to implement one or more configurations described herein. For example, the medical device 14 can be an external or noninvasive medical device, e.g., the device 14 configured to be located substantially external to the patient. For example, the therapeutic medical device 14, shown in FIG. 1 as a wearable defibrillator 14, as described herein can be bodily-attached to the patient such as the LifeVest^{®} wearable cardioverter defibrillator available from ZOLL^{®} Medical Corporation of Pittsburgh, PA and Chelmsford, MA. The wearable defibrillator 14 can be worn or carried by an ambulatory patient P. According to one example of the present disclosure, the wearable defibrillator 14 is used as an ambulatory cardiac monitoring and treatment device within a monitoring and treatment system according to the present disclosure.

Such wearable defibrillators can be typically worn nearly continuously for two to three months at a time. During the period of time in which they are worn by the patient, the wearable defibrillator 14 can be configured to continuously monitor the vital signs of the patient, to be user-friendly and accessible, to be as light-weight, comfortable, and portable as possible, and to be capable of delivering one or more life-saving therapeutic shocks when needed. Non-limiting examples of suitable ambulatory, wearable defibrillators are disclosed in United States Patent Nos. 4,928,690; 5,078,134; 5,741,306; 5,944,669; 6,065,154; 6,280,461; and 8,369,944.

The monitoring and treatment system illustrated in FIG. 1 includes the ambulatory, wearable defibrillator 14 connected to an electrode assembly 10 comprised of a number of monitoring/sensing electrodes 12 worn by the patient P so as to be in contact with the patient's skin. According to one example, the monitoring/sensing electrodes 12 can be configured to receive ECG signals from the patient P. The monitoring and treatment system illustrated in FIG. 1 also includes two or three therapy electrodes 11 worn by the patient P on his/her front and back so as to be in contact with the patient's skin. The therapy electrodes 11 can be configured to deliver one or more life-saving therapeutic shocks when needed. Prior to delivering the shock, one or more conductive gel deployment mechanisms may be deployed to cause conductive gel to be applied between the electrode(s) 11 and the patient's skin. In one implementation, a conductive gel deployment mechanism may be included in each therapy electrode 11 and the conductive gel may be deployed to the patient's skin via perforations in a conductive plate portion of the therapy electrode 11. The sensing electrodes 12 and the therapy electrodes 11 may be mutually connected by wires to form a single electrode assembly 10 that is connected to the wearable defibrillator 14. The electrode assembly 10 may further incorporate a vibration box 13 connected by wires to the sensing electrodes 12, the therapy electrodes 11, and to the wearable defibrillator 14. The vibration box 13 may be utilized to provide the patient P with a tactile alarm that the sensing electrodes 12 and/or the therapy electrodes 11 are not in proper contact with the patient's skin.

A patient being monitored by an in-hospital defibrillator and/or pacing device may be confined to a hospital bed or room for a significant amount of time (e.g., 90% or more of the patient's stay in the hospital). As a result, a user interface can be configured to interact with a user other than the patient, e.g., a nurse, for device-related functions such as initial device baselining, setting and adjusting patient parameters, and changing the device batteries.

In implementations, an example of a therapeutic medical device can include a shortterm continuous monitoring defibrillator and/or pacing device, for example, a short-term outpatient ambulatory, wearable defibrillator. For example, such a short-term outpatient wearable defibrillator can be prescribed by a physician for patients presenting with syncope. A wearable defibrillator can be configured to monitor patients presenting with syncope by, e.g., analyzing the patient's cardiac activity for aberrant patterns that can indicate abnormal physiological function. For example, such aberrant patterns can occur prior to, during, or after the onset of symptoms. In such an example implementation of the short-term wearable defibrillator, the electrode assembly can be adhesively attached to or otherwise supported against the patient's skin and have a similar configuration as the in-hospital defibrillator described above.

For example, the therapeutic medical devices described above (e.g., the wearable defibrillator, the in-hospital defibrillator, and the short-term defibrillator) can protect patients at risk for sudden cardiac death. It can be used for a wide range of patient conditions or situations, including following a recent myocardial infarction or coronary revascularization. In one scenario, the ambulatory, wearable defibrillator can give caregivers time to optimize medical therapy and assess a patient's long-term risk for sudden death. The wearable defibrillator is configured to continuously monitor the patient's heart and, if a life-threatening heart rhythm is detected, the device can issue an alert to the patient. For example, a monitor can include a touchscreen for programming as well as patient interaction, and response buttons for the patient to use when responding to treatment alerts. If the patient does not respond (e.g., by holding down the response buttons) the wearable defibrillator can deliver a treatment shock to restore normal heart rhythm.

### Exemplary Support Garment:

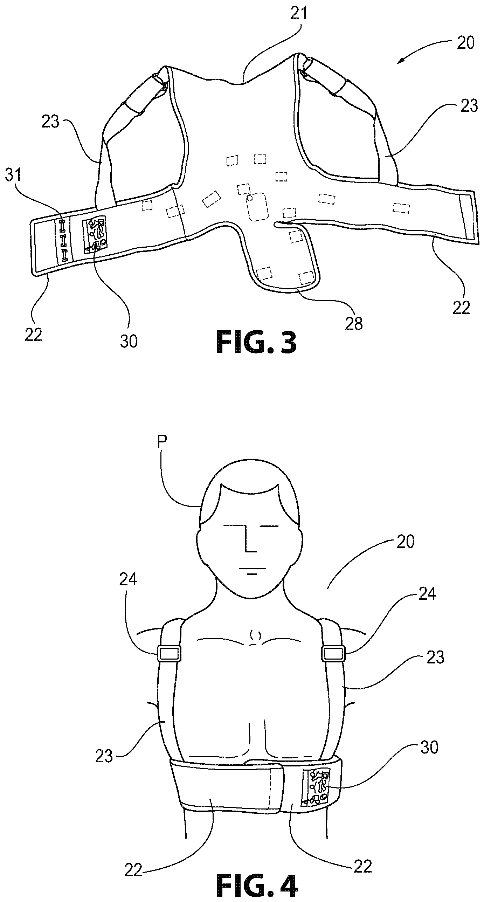

In accordance with one or more examples, a support garment 20 is provided to keep the electrodes 11, 12 in place against the patient's body while remaining comfortable during wear. FIGS. 2-5 illustrate such a support garment 20 in accordance with an example of the present disclosure. Such an exemplary support garment is described in United States Patent Application Publication No. 2012/0283794.

In order to obtain a reliable ECG signal so that the monitor can function effectively and reliably, the sensing electrodes must be in the proper position and in good contact with the patient's skin. The electrodes need to remain in a certain position, and not move excessively or lift off the skin's surface. If there is movement or lifting, the ECG signal will be adversely affected with noise and can cause problems with the detection system and in the monitoring system. Similarly, in order to effectively deliver the defibrillating energy, the therapy electrodes must be in the proper position and in good contact with the patient's skin. If the therapy electrodes are not firmly positioned against the skin, there can be problems with high impedance, leading to a less effective delivery of energy. If the therapy electrodes are not firmly positioned, there can also be damage to the patient's skin, such as burning, when the shock is delivered.

In accordance with one or more examples, the support garment 20 may provide comfort and functionality under circumstances of human body dynamics, such as bending, twisting, rotation of the upper thorax, semi-reclining, and lying down. These are also positions that a patient may assume if he/she were to become unconscious due to an arrhythmic episode. The design of the garment is generally such that it minimizes bulk, weight and undesired concentrations of force or pressure, while providing the necessary radial forces upon the treatment and sensing electrodes 11, 12 to ensure device functionality. The wearable defibrillator 14 may be disposed in a support holster (not shown) operatively connected to or separate from the support garment 20. The support holster may be incorporated in a band or belt worn about the patient's waist or thigh.

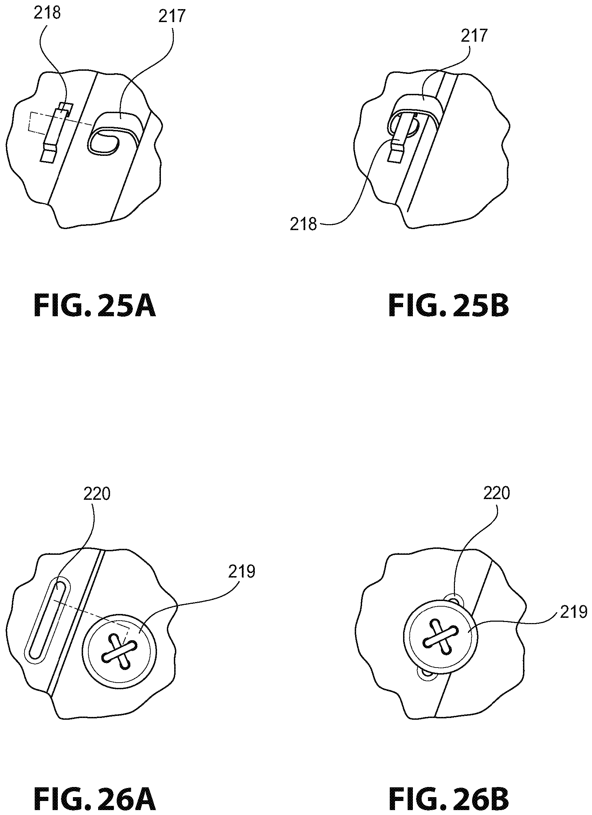

As shown in FIGS. 2-5, the support garment 20 may be provided in the form of a vest or harness having back portion 21 and sides extending around the front of the patient P to form a belt 22. The ends of the belt 22 are connected at the front of the patient P by a closure 31, which may comprise one or more clasps. Multiple corresponding closures 31 may be provided along the length of the belt 22 to allow for adjustment in the size of the secured belt 22 in order to provide a more customized fit to the patient P. An exemplary clasp closure is illustrated in FIGS. 25A and 25B and comprises a hook 217 disposed on one side to be connected and a corresponding eye 218 disposed on the opposing side to be connected. It is to be appreciated that the closure 31 may be of any type known to be suitable to those having ordinary skill in the art, such as a zipper, corresponding hook and pile fasteners, buttons, or snaps. The support garment 20 may further include two straps 23 connecting the back portion 21 to the belt 22 at the front of the patient P. The straps 23 have an adjustable size to provide a more customized fit to the patient P. The straps 23 may be provided with sliders 24 to allow for the size adjustment of the straps 23. The straps 23 may also be selectively attached to the belt 22 at the front of the patient P. The support garment 20 may be comprised of an elastic, low spring rate material that stretches appropriately to keep the electrodes 11, 12 in place against the patient's skin while the patient moves and is lightweight and breathable. For example, the support garment 20 may have elastic, low spring rate material composition based on a fiber content of about 20% elastic fiber, 32% polyester fiber, and up to 48% or more of nylon or other fiber. Appropriate materials for the support garment 20 are discussed in detail in the above-mentioned U.S. Patent Application Publication No. 2012/0283794.

In accordance with one or more examples, the support garment 20 may be formed from an elastic, low spring rate material and constructed using tolerances that are considerably closer than those customarily used in garments. The materials for construction are chosen for functionality, comfort, and biocompatibility. The materials may be configured to wick perspiration from the skin. The support garment 20 may be formed from one or more blends of nylon, polyester, and spandex fabric material. Different portions or components of the support garment 20 may be formed from different material blends depending on the desired flexibility and stretchability of the support garment 20 and/or its specific portions or components. For instance, the belt 22 of the support garment 20 may be formed to be more stretchable than the back portion 21. According to one example, the support garment 20 is formed from a blend of nylon and spandex materials, such as a blend of 77% nylon and 23% spandex. According to another example, the support garment 20 is formed from a blend of nylon, polyester, and spandex materials, such as 40% nylon, 32% polyester, and 14% spandex. According to another example, the support garment 20 is formed from a blend of polyester and spandex materials, such as 86% polyester and 14% spandex or 80% polyester and 20% spandex. Stitching within the support garment 20 may be made with industrial stitching thread. According to one example, the stitching within the support garment 20 is formed from a cotton wrapped polyester core thread.

The support garment 20 shown in FIGS. 2-5 may be configured for one-sided assembly of the electrode assembly 10 onto the support garment 20 such that the garment 20 does not need to be flipped or turned over in order to properly position the therapy electrodes 11 and the sensing electrodes 12 on the garment 20. The inside surface of the back portion support garment 20 includes pocket(s) 25 for receiving one or two therapy electrodes 11 to hold the electrode(s) 11 in position against the patient's back. The pocket 25 is made from a non-elastic, conductive mesh fabric designed to isolate the metallic therapy electrode(s) 11 from the skin of the patient P while allowing a conductive gel that may be automatically extruded from the electrode(s) 11 to easily pass through. The forces applied to the electrode(s) 11 by the fabric, in addition to the use of the conductive gel, may help ensure that proper contact and electrical conductivity with the patient's body are maintained, even during body motions. The fabric material of the pocket(s) 25 also maintains electrical contact between the electrode(s) 11 through the mesh material before the conductive gel is dispensed, which allows for monitoring of the therapy electrode(s) 11 to ensure that the electrode(s) are positioned against the skin such that a warning may be provided by the wearable defibrillator 14 if the therapy electrode(s) 11 is not properly positioned. Another pocket 26 made from the same non-elastic, conductive mesh fabric is included on an inside surface of the belt 22 for receiving a therapy electrode 11 and holding the electrode 11 in position against the patient's left side. According to one example, the pockets 25, 26 are formed from a knit electrically conductive material. The material of the pockets 25, 26 may have a metal coating, such as a silver coating, applied thereto to provide electrical conductivity. The pockets 25, 26 may be closed by conventional fasteners, such as snaps, buttons or hook and pile fasteners.

In other examples, instead of being removably disposed within pocket(s) 25, 26, the electrode(s) 11 may be permanently integrated in a non-removable fashion into the support garment 20. In some implementations involving electrode(s) 11 that are permanently integrated into the support garment 20, conductive gel may be stored in one or more removably attached receptacles that are disposed in proximity to each electrode(s) 11. Such exemplary support garments having permanently integrated electrodes and removably attached conductive gel receptacles are described in United States Patent Application Publication No. 2016/0022982.

The back portion 21 and the belt 22 of the support garment 20 may further incorporate attachment points 27 for supporting the sensing electrodes 12 in positions against the patient's skin in spaced locations around the circumference of the patient's chest. The attachment points 27 may include hook and pile fasteners for attaching electrodes 12 having a corresponding fastener disposed thereon to the inside surface of the belt 22. The attachment points 27 may be color coded to provide guidance for appropriately connecting the sensing electrodes 12 to the support garment 20. The support garment 20 may further be provided with a flap 28 extending from the back portion 21. The flap 28 and the back portion 21 include snap fasteners 29 for connecting the flap 28 to the inside surface of the back portion 21 in order to define a pouch or pocket for holding the vibration box 13. The outer surface of the belt 22 may incorporate a schematic 30 imprinted on the fabric for assisting the patient or medical professional in assembling the electrode assembly 10 onto the support garment 20.

In an alternative exemplary support garment, the back portion includes a pocket accessible from an outer side of the garment for receiving the therapy electrode(s). A layer of the mesh material may form the inner surface of the back portion to allow the conductive gel to pass therethrough to contact the patient's skin. The pocket may be closed with snap fasteners. The back portion also includes an external holster pocket for receiving the vibration box therein. A strap is provided on the back portion for securing the vibration box in the external holster. Another holster or pocket is provided on the inner surface of the belt on the patient's left side for receiving another therapy electrode. The pocket is accessible from the inside surface of the belt and may include a snap enclosure for securing the therapy electrode. The pocket includes a layer of the mesh material discussed above to allow the conductive gel to pass through to the patient's skin. The inside surface of the back portion and the belt also include attachment points, as discussed above, for connection of the sensing electrodes to the support garment. Accordingly, in order to assemble the electrode assembly onto the support garment, the support garment must be turned over to access the pocket and holster on the back portion and subsequently the pocket on the belt and the attachment points. The support garment according to this example may include three straps. Two straps extend from the top of the back portion over the patient's shoulders and under the patient's arms to connect to points on the back portion below the pocket for the therapy electrode(s). A third strap extends from the left shoulder strap at a point near the patient's shoulder to the left side of the belt proximate to the pocket on the belt for receiving the therapy electrode. The straps may be selectively attached to the back portion and the belt and may have an adjustable size.

### Overview of Support Garment According to the Present Disclosure:

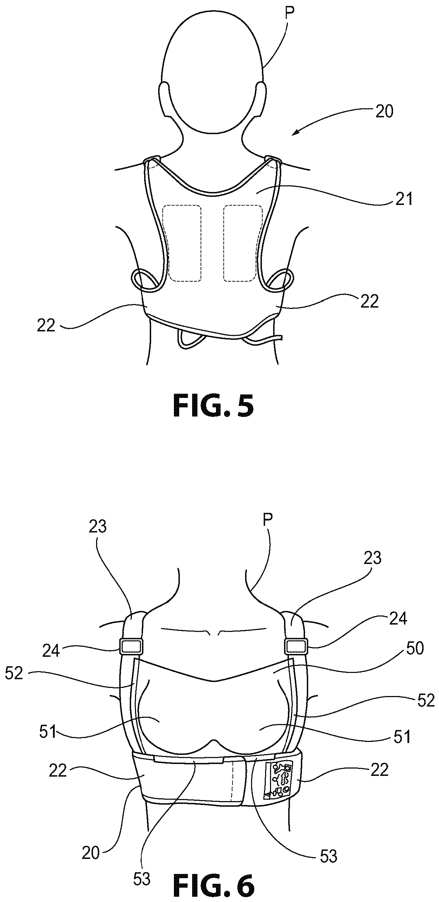

FIGS. 6-21 illustrate various examples of a support garment according to the present disclosure. The illustrated examples of the support garment each include a bra or bra portion configured to support front upper torsal female anatomy. In particular, each of the illustrated support garments 0 includes two cups configured to contact and support the patient's breasts.

During use of the support garment 20 discussed above with reference to FIGS. 2-5, female patients are advised to put on the garment 20 before putting on a bra or similar garment that provides support to their breasts such that the garment 20 is positioned underneath the patient's bra. However, this arrangement is uncomfortable because the garment straps 23 and fabric interfere with the fit and comfort of the bra. Further, the bra is still likely to interfere with the position of the support garment 20, and thereby the position of the sensing electrodes 12 and the therapy electrodes 11, on the patient.

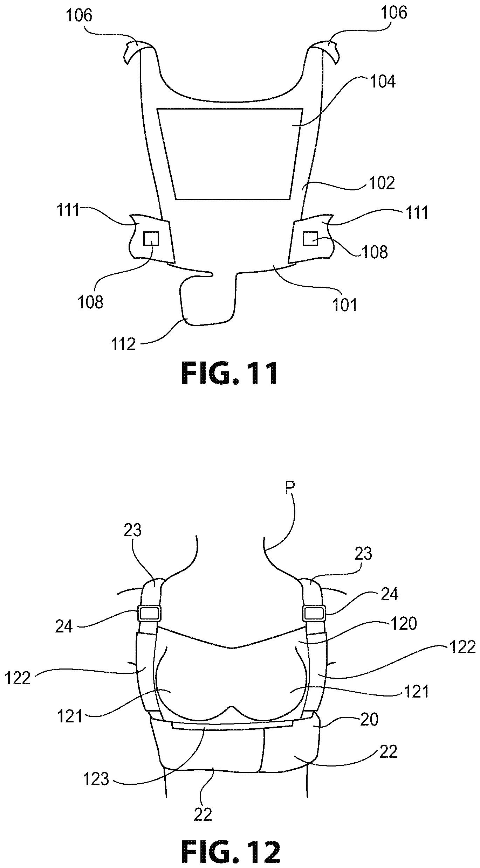

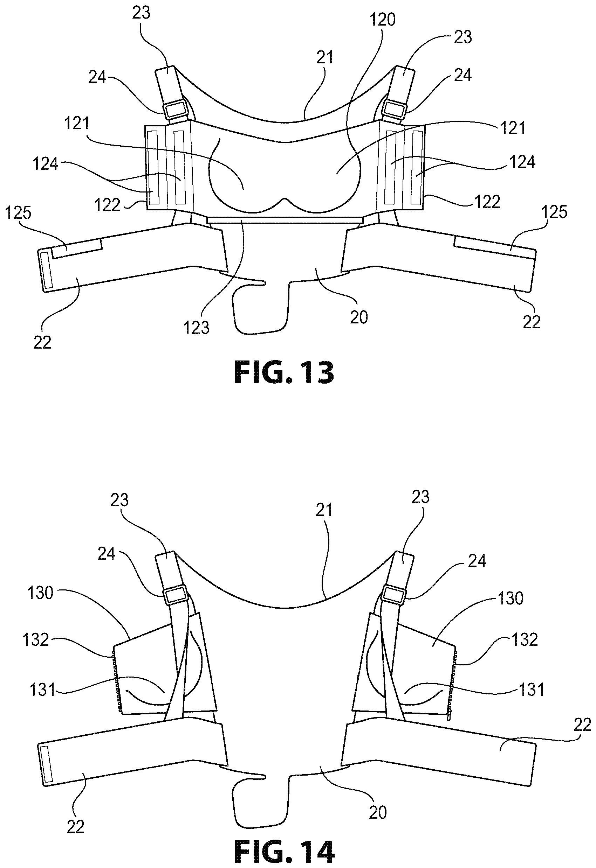

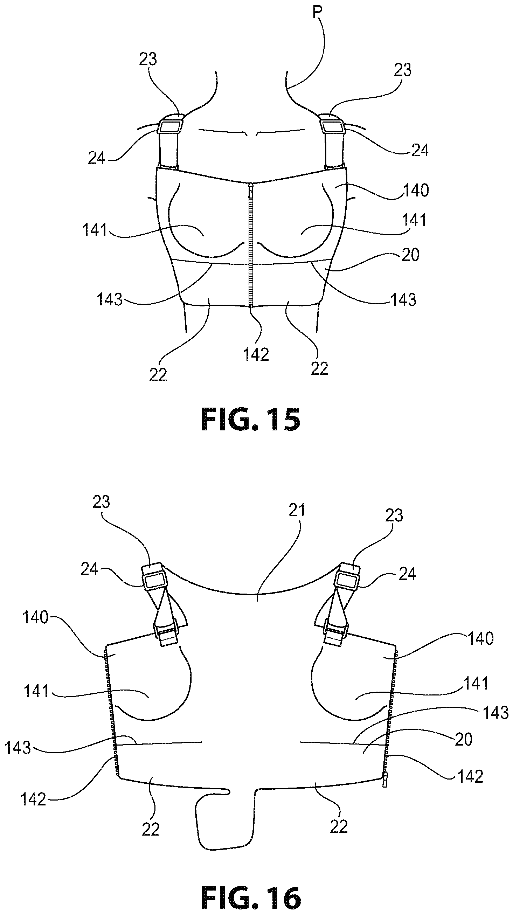

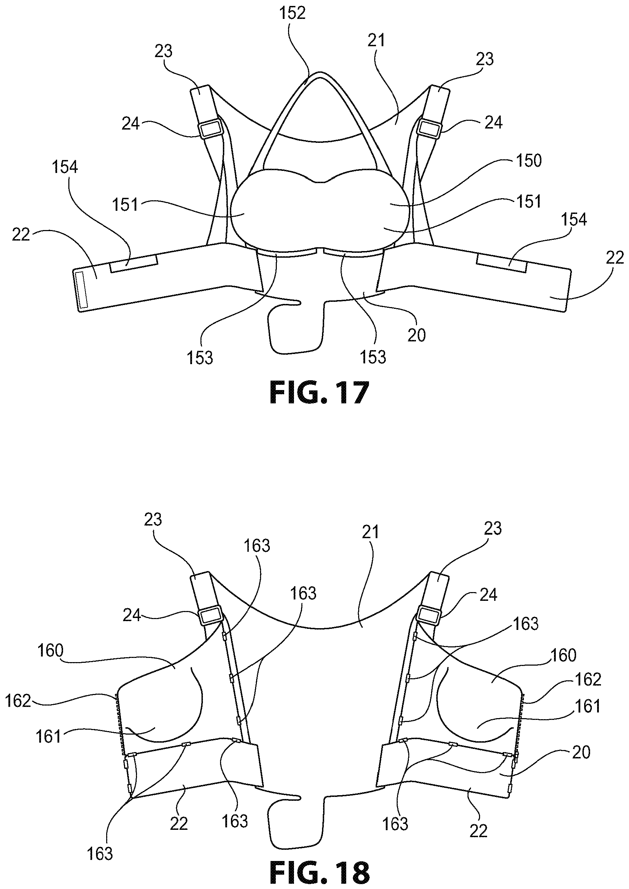

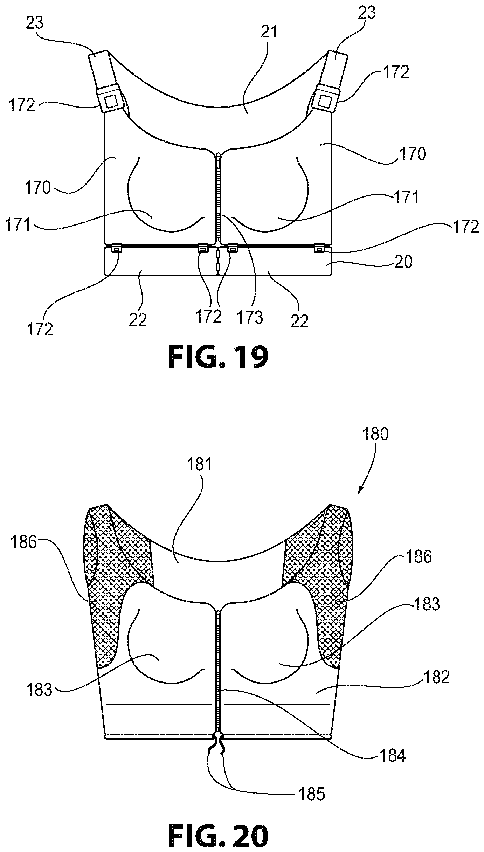

The examples illustrated with respect to FIGS. 6-21 provide multiple solutions to the problems found with the support garment 20. The examples illustrated with respect to FIGS. 6, 7, and 12-19 provide female patients with a support bra attachment portion or similar attachment garment that attaches to the existing support garment 20 discussed above in order to provide female patients more comfortable support for their breasts while wearing the support garment 20. As illustrated in these examples, the bra structure may be in the form of a bra attachment portion(s) or breast plate 50 (FIG. 6), 120 (FIG. 12), 130 (FIG. 14), 140 (FIG. 15), 150 (FIG. 17), 160 (FIG. 18), 170 (FIG. 19) that connects to at least one of the shoulder straps 23, the belt 22, and the back portion 21 of the support garment 20. As illustrated, various garment structures and attachment mechanisms may be utilized in connection with these examples. The bra attachment portion(s) or breast plates may be provided in different sizes and styles to accommodate different body types and breast sizes. The bra attachment portion(s) or breast plates may be provided with adjustable support, as will be discussed in further detail.

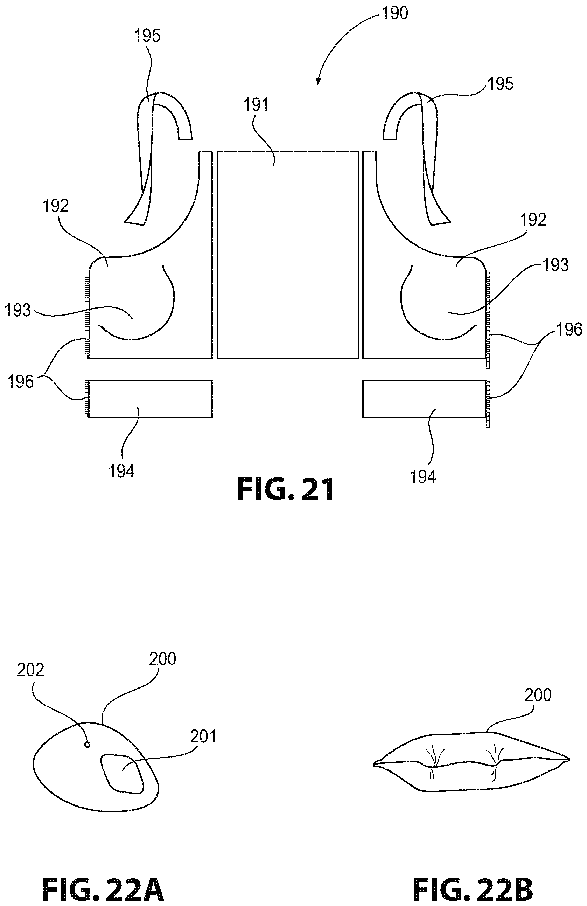

The examples illustrated with respect to FIGS. 8-11, 20, and 21 provide female patients with a support garment 100 (FIG. 8), 180 (FIG. 20), 190 (FIG. 21) that is formed as a bra or incorporates a bra structure and incorporates one or more support members and attachment points on the inside surface thereof for supporting the electrode assembly 10 discussed above with reference to FIGS. 1 and 2 on the support garment 100, 180, 190 such that the sensing electrodes 12 and the therapy electrode(s) 11 may be positioned against the patient's skin at the proper locations on the patient's body. The support garments 100, 180, 190 according to these examples are provided as an alternative garment for the support garment 20 discussed above. The support garments 100, 180, 190 incorporate cups for contacting and supporting the patient's breasts in a more comfortable manner. The support garments 100, 180, 190 may be provided in different sizes and styles to accommodate different body types and breast sizes. The cups may be provided with adjustable support, as will be discussed in further detail.

The support garments according to the present disclosure offer several improvements/advantages over the support garment 20, discussed above, and other similar support garments known in the art. As stated above, the support garment 20, as well as other similar garments, is intended to be worn under the patient's bra, but this is very uncomfortable because the support garment 20 interferes with the bra and the bra causes the garment 20 to dig into the skin, especially the straps. As such, female patients tend to wear their bra under the support garment 20, which results in the bra interfering with the positioning of the electrodes against the patient's skin.

The support garments illustrated in FIGS. 6-21 eliminates the need for patients to wear a bra separate from the support garment, which as discussed above, can possibly be worn in a manner that interferes with the electrodes. The support garments illustrated in FIGS. 6-21 combine the bra structure into the support garment for supporting and holding the electrodes in the proper positions against the patient's skin to form a single structure so that both the bra and the support garment can be worn properly by the patient and move together as the patient moves.

The support garments illustrated in FIGS. 6-21 improves the comfort and styling of the therapeutic garment for female patients. This is accomplished by eliminating/reducing multiple layers of fabric worn by the patient and reducing the number of over the shoulder straps and other elastic elements to be worn by the patient. Further, the support garments illustrated in FIGS. 6-21 may incorporate any structural support and styling features typically used in bras subject to the requirements of supporting the electrode assembly and holding the electrodes against the patient's skin in the proper locations on the patient's body.

The patient's bra and the support garment 20 tend to occupy the same space on the patient's front below the breasts and the support garment 20 will ride up over the bra regardless of whether the bra is worn under or over the support garment 20. The support garments illustrated in FIGS. 6-21 eliminate this problem by using the structure of the support garment as the base structure of the bra.

The support garments illustrated in FIGS. 6-21 eliminates the redundant structures of the bra and the support garment 20, such as the straps, back, and the front support structure for the cups by incorporating the bra structure into the support garment or by incorporating the electrode assembly 10 into a suitably configured bra or similar garment.

### Support Garment Incorporating a Breast Plate:

FIGS. 6 and 7 illustrate a support garment for supporting a patient wearable defibrillator according to an example of the present disclosure. The illustrated support garment is a combination of the support garment 20 discussed above with reference to FIGS. 2-5 and a bra attachment portion 50 in the form of a breast plate. As discussed above, the support garment 20 is made from a fabric having an outside surface and an inside surface and is configured to be worn about a chest of a patient P. The support garment 20 comprises a back portion 21, a belt 22 defined by side portions extending from the back portion 21 around a front of the patient P, and shoulder straps 23 attached to the back portion 21 and the belt 22. The back portion 21 and the belt 22 are configured to support a plurality of sensing electrodes 12, shown in FIGS. 1 and 2, and at least one therapeutic defibrillator electrode 11, also shown in FIGS. 1 and 2.

The bra attachment portion 50 is configured to support front upper torsal female anatomy and extends between and is connected to the straps 23 and the belt 22 of the support garment 20 at the front of the patient P. The bra attachment portion 50 includes two cups 51 that are configured to contact and support the patient's breasts. The cups 51 incorporate suitable structure for supporting the patient's breasts in a comfortable and stylized manner that is typical of various bra garments known in the art. Accordingly, the cups 51 may include layers of foam padding, underwire, and/or plastic inserts typically used in bra garments to provide comfortable support for the patient's breasts and a suitable fit for the patient. The cups 51 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portion 50 may be worn under the patient's normal clothing in a manner that minimizes noticeable protrusion of the bra attachment portion 50 and interference with the fit and comfort of the patient's clothing. In other words, the bra attachment portion 50 is structured to fit closely to the patient's body and to conform to the shape of the patient's front upper torsal anatomy such that the outward extension of the bra attachment portion 50 from the patient's body is minimized so that the bra attachment portion 50 does not extend or only minimally extends into a space generally occupied by the patient's normal clothing.

The bra attachment portion 50 includes fastening mechanisms 52, 53 for connecting the bra attachment portion 50 to the belt 22 and the straps 23 of the support garment 20. The support garment 20 includes corresponding fastener mechanisms 54 on the belt 22 and on the straps 23. According to one example of the disclosure, the fastening mechanisms 52, 53, 54 include corresponding hook and pile fasteners provided on the sides and bottom of the bra attachment portion 50 and along the top inside surface of the belt 22 and on the straps.

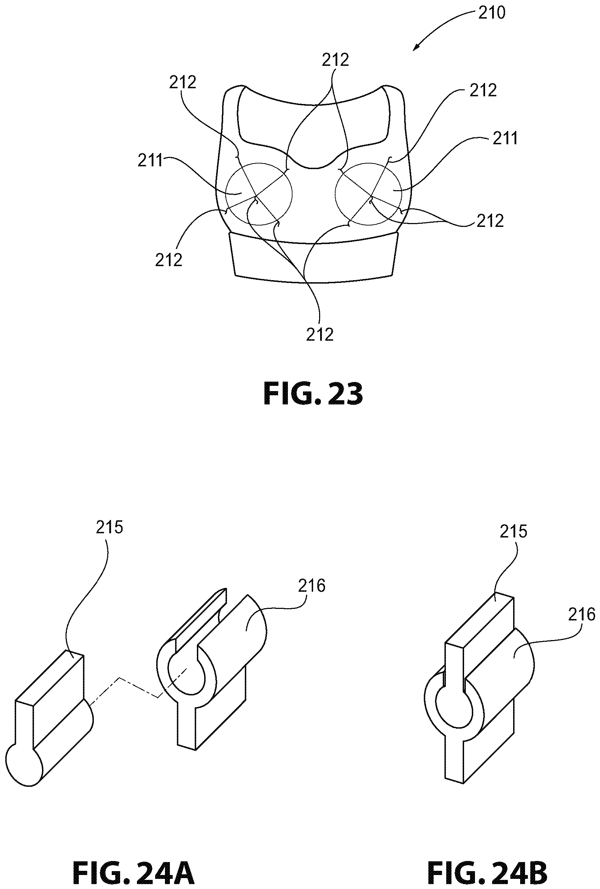

According to another example of the disclosure illustrated in FIGS. 24A and 24B, the fastening mechanisms 52, 53, 54 include corresponding plastic male and female mateable slide connectors 215, 216. According to this example, the bra attachment portion 50 is provided with a male or female connector 215, 216 on each of its lateral sides and along its bottom. The belt 22 and the straps 23 are provided with corresponding male or female connectors 215, 216. The bra attachment portion 50 is connected to the support garment 20 by sliding the male connectors 215 into and along the corresponding female connectors 216 until the bra attachment portion 50 is properly connected and positioned on the support garment 20. The male and female slide connectors 215, 216 may be attached by stitching or adhered to the bra attachment portion 50 and to the belt 22 and straps 23 of the support garment 20. The mateable slide connectors 215, 216 are useful to integrate the bra attachment portion 50 more fully into the structure of the support garment 20 so that the bra attachment portion 50 is more secure.

According to the example of the disclosure illustrated in FIGS. 25A and 25B, the bra attachment portion 50 is connected to the belt 22 and/or the straps 23 of the support garment 20 by a plurality of clasps provided on the lateral sides and the bottom of the bra attachment portion 50 and on the belt 22 and the straps 23. Each clasp mechanism includes a hook 217 provided on one of the bra attachment portion 50 and the support garment 20 and a corresponding eye 218 provided on the other of the bra attachment portion 50 and the support garment 20. The bra attachment portion 50 is secured on the support garment 20 by sliding the hooks 217 into the corresponding eyes 218.

According to the example of the disclosure illustrated in FIGS. 26A and 26B, the bra attachment portion 50 is connected to the belt 22 and/or the straps 23 of the support garment 20 by a plurality of buttons 219 and corresponding button holes 220 provided on the lateral sides and the bottom of the bra attachment portion 50 and on the belt 22 and the straps 23. The bra attachment portion 50 is secured by inserting the buttons 219 on one of the bra attachment portion 50 and the support garment 20 into the corresponding button holes 220 provided on the other of the bra attachment portion 50 and the support garment 20.

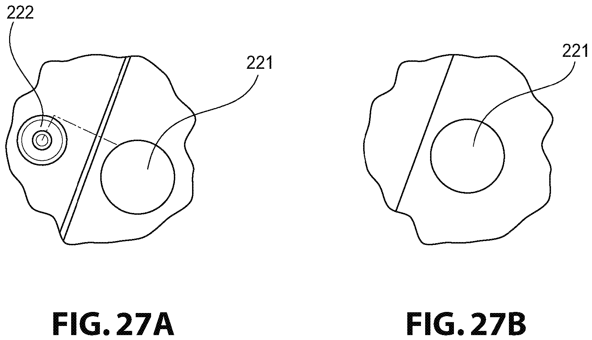

According to the example of the disclosure illustrated in FIGS. 27A and 27B, the bra attachment portion 50 is connected to the belt 22 and/or the straps 23 of the support garment 20 by a plurality of snaps each comprised of female snap 221 and a male snap 222. The corresponding snaps 221, 222 are provided on the lateral sides and the bottom of the bra attachment portion 50 and on the belt 22 and the straps 23. The bra attachment portion 50 is secured by inserting the male snaps 222 into the corresponding female snaps 221.

According to another example of the disclosure, the bra attachment portion 50 is connected to the belt 22 and/or the straps 23 by press fit connectors 163, which are illustrated in FIG. 18, or by mateable buckles 172, which are illustrated in FIG. 19. It is to be appreciated that any fastening mechanism or mechanisms, including combinations of the fastening mechanisms discussed above, may be used to secure the bra attachment portion 50 to the belt 22 and the straps 23 of the support garment 20.

With reference to FIGS. 6 and 7, the bra attachment portion 50 may be formed from any material or materials known to be suitable to those having ordinary skill in the art. In particular, the bra attachment portion 50 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portion 50 may be provided in multiple sizes and with multiple cup sizes to fit patients of different body types. For instance, the bra attachment portion 50 may be provided in multiple sizes S, M, L, XL, XXL, etc. corresponding to the size of the support garment 20. The bra attachment portion 50 may also be provided having different sized cups A, B, C, D, etc. typically found in bra garments. The bra attachment portion 50 may also include an adjustment mechanism configured to adjust a fit of the bra attachment portion 50 to the patient P. The adjustment mechanism is configured to provide a more customizable fit of the bra attachment portion 50 to the patient P. The inclusion of the adjustment mechanism in the bra attachment portion 50 may also allow for a reduction in the number of specific cup sizes provided for the bra attachment portion 50 at each of the multiple sizes in order to reduce the manufacturing and inventory costs associated with manufacturing and managing inventory for bra attachment portions 50 at each particular cup size used for bra garments.

With reference to FIGS. 22A and 22B, in accordance with one example the adjustment mechanism comprises an inflatable insert 200 that may be inserted into each of the cups 51 of the bra attachment portion 50. The size of the inflatable insert 200 may be increased by pressing the pump button 201 on the top side of the insert 200 to inflate the insert 200 and may be decreased by pressing the release button 202 on the top side of the insert 200 to release air from the insert 200. The inflatable insert 200 may be placed into the cups 51 separately by the patient P or permanently attached or adhered to the bra attachment portion 50 during manufacturing. The inflatable insert 200 may also be used to adjust the styling of the bra attachment portion 50.

With reference to FIG. 23, in accordance with another example the adjustment mechanism comprises a plurality of drawstrings 212. As illustrated, the drawstrings 212 are incorporated into the cups 211 of a support garment 210 according to any one of the examples of FIGS. 6-21. Each cup 211 may be provided with five drawstrings 212 incorporated into the structure of the cup 211 and extending from the center of the cup 211. Four of the drawstrings 212 extend diagonally from the center of the cup 211 to the outside of the cut 211. A fifth drawstring 212 extends from the center of the cup 211. The cups 211 are structured to allow for changes in the sizing of the cups 211. During fitting of the support garment 210, the drawstrings 212 may be drawn to decrease the size of the cups 211 to provide the patient P with a customized fit. When the fit is completed, the ends of the drawstrings 212 may be cut and tied off or otherwise secured to maintain the size of the cups 211. Alternatively, the sizing of the cups 211 may remain adjustable by providing suitable clamps on the drawstrings 212.

The bra attachment portion 50 illustrated in FIGS. 6 and 7 is advantageous because it requires minimal modification to the existing support garment 20 so that different sized support garments 20 may be matched with different sized bra attachment portions 50 at the time of fitting and utilizes existing support garments 20 to supplement the structure of the bra attachment portion 50 to form a bra. When the male and female plastic slide connectors 216, 217 are utilized to connect the bra attachment portion 50 to the support garment 20, the plastic slide connectors 216, 217 integrate the bra attachment portion 50 more fully into the structure of the support garment 20 so that the bra attachment portion 50 is more secure.

### Bra Garment Incorporating Support Structure for a Patient Wearable Defibrillator

FIGS. 8-11 illustrate a patient wearable defibrillator 100 that includes a support garment 101 in the form of a bra or similar garment, such as a tank top, halter top, or camisole, according to an example of the present disclosure. In some embodiments, the treatment components (e.g., the therapeutic defibrillator electrode 11 and associated circuitry) may be optional, e.g., such components may be removably attached to the support garment 101. Accordingly, while the disclosure herein refers to a patient wearable defibrillator 100, it is understood that the device may be in the form of a cardiac monitor as described above, and include optional, removably attached defibrillation and/or treatment elements.

A defibrillator 100 includes an electrode assembly 10 having a plurality of sensing electrodes 12 configured to monitor a cardiac function of the patient P and, if provided, at least one therapeutic defibrillator electrode 11, as discussed above with reference to FIGS. 1 and 2. In addition, as noted above, the defibrillator 100 can be configured to monitor other physiologic parameters of the patient in addition to cardiac related parameters. For example, the device can be configured to monitor, for example, lung sounds (e.g., using microphones and/or accelerometers), breath sounds, respiration, sleep related parameters (e.g., snoring, sleep apnea), tissue fluids (e.g., using radio-frequency transmitters and sensors), among others.

For example, referring to FIG. 8, an acoustic sensor 59 may be provided to monitor one or more of a patient's lung sounds, heart sounds, breath sounds, respiration, and/or other sounds produced within the patient's body. For example, the acoustic sensor 59 may be configured to determine fiducial timepoints in the mechanical activity of the heart (such as detection and monitoring of S1, S2, S3, and S4 sounds) as exemplified by AUDICOR^{®} Technology from Inovise Medical of Beaverton, Oregon.

For example, referring again to FIG. 8, a fluid monitor 60 may be provided to monitor one or more of a patient's lung and/or other tissue fluid level, e.g., using ultra wide band (UWB) radio frequency technology.

The support garment 101 is made from a fabric having an outside surface and an inside surface and is configured to be worn about a chest of the patient P. The support garment 101 is configured to support and hold the sensing electrodes 12 and the at least one therapeutic defibrillator 11 against the patient's skin at the proper locations on the patient's body in a manner similar to the support garment 20 discussed above with reference to FIGS. 2-5.

According to one example, the support garment 101 may be formed from a blend of nylon and spandex materials, such as a blend of 77% nylon and 23% spandex. According to another example, the support garment 101 is formed from a blend of nylon, polyester, and spandex materials, such as 40% nylon, 32% polyester, and 14% spandex. According to another example, the support garment 20 is formed from a blend of polyester and spandex materials, such as 86% polyester and 14% spandex or 80% polyester and 20% spandex. The material used in the support garment 101 may vary depending on the portion of the garment and the part of the anatomy that the garment comes in contact with. For example, the portion of the garment that comes in contact with the front upper torsal female anatomy as described below may use different material than the rest of the support garment 101. Stitching within the support garment 101 may be made with industrial stitching thread. According to one example, the stitching within the support garment 101 is formed from a cotton wrapped polyester core thread. Further, the support garment 101 may be based on a low elasticity, low spring rate material. For example, the fiber content of such a low spring rate material may be about 20% elastic fiber, 32% polyester fiber, and up to 48% or more of nylon or other fiber.

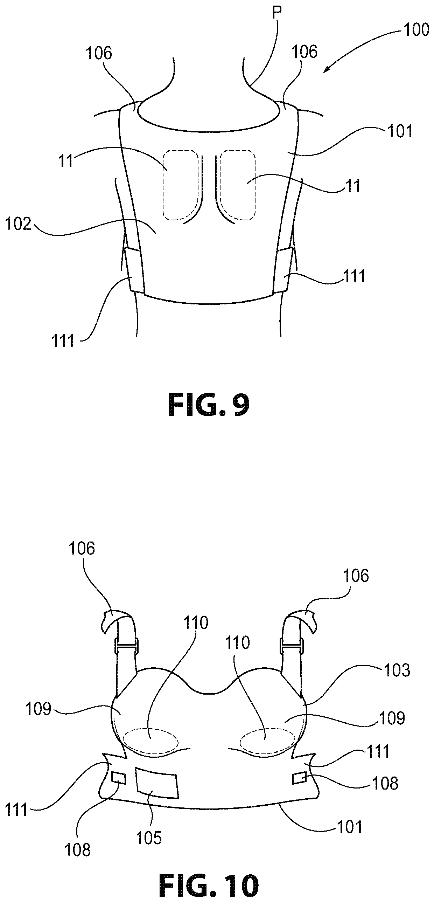

As shown in FIGS. 8 and 9, the support garment 101 includes a back portion 102 and a front portion 103 connected to the back portion 102 by sides 111. The front portion 103 of the garment 101 is configured to support front upper torsal female anatomy. The support garment 101 also includes shoulder straps 106 connecting the front portion 103 to the back portion 102. The shoulder straps 106 may be provided with sliders 107 so that the size of the shoulder straps 106 can be adjusted.

As shown in FIGS. 10 and 11, the sensing electrodes 12 and the at least one therapeutic defibrillator electrode 11 are supported on the inside surface of the support garment 101. The support garment 101 includes at least one support member 104, 105 on the inside surface thereof for supporting the at least one therapeutic defibrillator electrode 11 on the support garment 101. In particular, the inside surface of the back portion 102 has a support member in the form of pocket(s) 104 defined thereon, which is configured to receive one or two therapy electrodes 11 therein in the same manner as the pocket(s) 25 discussed above with reference to FIGS. 2-5. The pocket(s) 104 includes a layer of conductive, mesh material disposed between the at least one therapy electrode 11 and the patient's skin to allow for the conductive gel to pass through the mesh material and into contact with the patient's skin and to allow for electrical connnection to be maintained between the therapy electrodes 11 and the patient's skin. Further, by detecting contact between the therapy electrodes 11 and the mesh material, the wearable defibrillator 14 is able to detect if the therapy electrodes 11 are not in proper contact with the patient. The inside surface of the front portion 103 also has a support member in the form of a pocket 105 defined thereon, which is configured to receive another therapy electrode therein in the same manner as the pocket 26 discussed above with reference to FIGS. 2-5. The pocket 105 also includes a layer of mesh material disposed between the therapy electrode 11 and the patient's skin. According to one example, the pockets 104, 105 are formed from a knit electrically conductive material. The material of the pockets 104, 105 may have a metal coating, such as a silver coating, applied thereto to provide electrical conductivity between the therapy electrodes 11 and the patient's skin. The support garment 101 also includes attachment points 108 on the inside surface thereof for attaching the sensing electrodes 12 to the inside surface of the support garment 101 and distributing the sensing electrodes 12 around the circumference of the patient's chest in a similar manner as the attachment points 27 discussed above with reference to FIGS. 2-5. The attachment points 108 may be similarly color coded for proper assembly of the sensing electrodes 12 onto the support garment 101. The support garment 101 may further include a flap 112 depending from the back portion 102 that can be connected to the back portion 102 to form a pouch or pocket for supporting a vibration box 13 of the electrode assembly 10 in the same manner as the flap 28 discussed above with reference to FIGS. 2-5.

The support garment 101 includes two cups 109 that are configured to contact and support the patient's breasts. The cups 109 incorporate suitable structure for supporting the patient's breasts in a comfortable and stylized manner that is typical of various bra garments known in the art. Accordingly, the cups 109 may include layers of foam padding, underwire, and/or plastic inserts typically used in bra garments to provide comfortable support for the patient's breasts and a suitable fit for the patient. The cups 109 may be structured, through the use of the above-mentioned layers of foam padding, underwire, and/or plastic inserts, in a smooth rounded shape to match the contours of the patient's front upper torsal anatomy and to define a smoothed, stylized silhouette such that the support garment 101 may be worn under the patient's normal clothing in a manner that minimizes noticeable protrusion of the support garment 101 and interference with the fit and comfort of the patient's clothing. In other words, the support garment 101 may be structured to fit closely to the patient's body and to conform to the shape of the patient's front upper torsal anatomy such that the outward extension of the support garment 101 from the patient's body is minimized so that the support garment 101 does not extend or only minimally extends into a space generally occupied by the patient's normal clothing. The support garment 101 may be split in the front and provided with a front closure mechanism, such as a zipper, a plurality of clasps or a similar fastener, to connect the separate halves of the front portion 103 at the front of the patient P in order to facilitate wearing and removing of the support garment 101.

The support garment 101 may be formed from any material or materials known to be suitable to those having ordinary skill in the art. In particular, the support garment 101 may be formed from the same elastic, low spring rate material as the support garment 20 discussed above with reference to FIGS. 2-5, such as a blend of spandex material with nylon and/or polyester materials. As also discussed above, the stitching may be made from a cotton wrapped pop[ polyester core thread. The support garment 101 may be provided in multiple sizes and with multiple cup sizes to fit patients of different body types. For instance, the support garment 101 may be provided in multiple sizes S, M, L, XL, XXL, etc. The support garment 101 may also be provided having different cup sizes A, B, C, D, etc. typically found in bra garments. Each cup 109 of the support garment 101 may also include an adjustment mechanism 110 configured to adjust a fit of the cup 109 to the patient P. The inclusion of the adjustment mechanisms 110 in the support garment 101 may also allow for a reduction in the number of specific cup sizes provided for the support garment 101 at each of the multiple sizes in order to reduce the manufacturing and inventory costs associated with manufacturing and managing inventory for support garments 101 at each particular cup size used for bra garments. The adjustment mechanism 110 may comprise an inflatable insert 200 as discussed above with reference to FIGS. 22A and 22B or a plurality of drawstrings 212 as discussed above with reference to FIG. 23.

The support garment 101 is advantageous because it fully integrates the support structures for the electrode assembly 10 found in the support garment 101 into a bra garment such that the bra garment can fulfill the function of the support garment 101 and provide similar comfort and styling features as a typical bra garment.

### Support Garment Incorporating a Bra Band:

FIGS. 12 and 13 illustrate a support garment for supporting a patient wearable defibrillator according to an example of the present disclosure. The illustrated support garment is a combination of the support garment 20 discussed above with reference to FIGS. 2-5 and a bra attachment portion 120 in the form of a bra band.

The bra attachment portion 120 is configured to support front upper torsal female anatomy and extends between and is connected to the straps 23 and the belt 22 of the support garment 20 at the front of the patient P. The bra attachment portion 120 includes two cups 121 that are configured to contact and support the patient's breasts. The cups 121 incorporate suitable structure for supporting the patient's breasts in a comfortable and stylized manner that is typical of various bra garments known in the art. Accordingly, the cups 121 may include layers of foam padding, underwire, and/or plastic inserts typically used in bra garments to provide comfortable support for the patient's breasts and a suitable fit for the patient. The cups 121 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portion 120 may be worn under the patient's normal clothing in a manner that minimizes noticeable protrusion of the bra attachment portion 120 and interference with the fit and comfort of the patient's clothing. In other words, the bra attachment portion 120 may be structured to fit closely to the patient's body and to conform to the shape of the patient's front upper torsal anatomy such that the outward extension of the bra attachment portion 120 from the patient's body is minimized so that the bra attachment portion 120 does not extend or only minimally extends into a space generally occupied by the patient's normal clothing.

The bra attachment portion 120 includes fastening flaps 122 on both lateral sides thereof. The flaps 122 are configured to wrap around the straps 23 of the support garment 20 and include fastening mechanisms 124 to secure the ends of the flaps 122 to the bra attachment portion 120 in order to connect the bra attachment portion 120 to the straps 23. According to an example of the present disclosure, the fastening mechanisms 124 comprise corresponding strips of hook and pile fasteners. It is to be appreciated that other fastening mechanisms, such as clasps, buttons, or snaps, may be used to secure the ends of the flaps 122 to the bra attachment portion 120.

The bra attachment portion 120 also includes a fastening mechanism 123 disposed along the bottom thereof for connecting the bra attachment portion 120 to the belt 22. The belt 22 includes corresponding fastening mechanisms 125. As discussed above with respect to FIGS. 6 and 7, the fastening mechanisms 123, 125 may comprise strips of hook and pile fasteners, plastic male and female mateable slide connectors 215, 216 of the type shown in FIGS. 24A and 24B, a plurality of clasps of the type shown in FIGS. 25A and 25B, a plurality of buttons of the type shown in FIGS. 26A and 26B, and/or a plurality of snaps of the type shown in FIGS. 27A and 27B. The bra attachment portion 120 may also be connected to the belt 22 by press fit connectors or by mateable buckles.

The bra attachment portion 120 may be formed from any material or materials known to be suitable to those having ordinary skill in the art. In particular, the bra attachment portion 120 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portion 120 may be provided in multiple sizes and with multiple cup sizes to fit patients of different body types. For instance, the bra attachment portion 120 may be provided in multiple sizes S, M, L, XL, XXL, etc. corresponding to the size of the support garment 20. The bra attachment portion 120 may also be provided having different sized cups A, B, C, D, etc. typically found in bra garments. The bra attachment portion 120 may also include an adjustment mechanism, such as the inflatable insert 200 shown in FIGS. 22A and 22B or the plurality of drawstrings 212 shown in FIG. 23, configured to adjust a fit of the bra attachment portion 120 to the patient P.

The bra attachment portion 120 illustrated in FIGS. 12 and 13 is advantageous because it requires minimal modification to the existing support garment 20 so that different sized support garments 20 may be matched with different sized bra attachment portions 120 at the time of fitting and utilizes existing support garments 20 to supplement the structure of the bra attachment portion 120 to form a bra.

### Support Garment Incorporating Bra attachment portions Connected to the Back Portion:

FIG. 14 illustrates a support garment for supporting a patient wearable defibrillator according to an example of the present disclosure. The illustrated support garment is a combination of the support garment 20 discussed above with reference to FIGS. 2-5 and two bra attachment portions 130 each comprising a cup 131 configured to contact and support one of the patient's breasts. As discussed above, the support garment 20 is made from a fabric having an outside surface and an inside surface and is configured to be worn about a chest of a patient P. The support garment 20 comprises a back portion 21, a belt 22 defined by side portions extending from the back portion 21 around a front of the patient P, and shoulder straps 23 attached to the back portion 21 and the belt 22. The back portion 21 and the belt 22 are configured to support a plurality of sensing electrodes 12, shown in FIGS. 1 and 2, and at least one therapeutic defibrillator electrode 11, also shown in FIGS. 1 and 2.

The two bra attachment portions 130 are connected by fasteners or by stitching to opposing sides of the back portion 21 of the support garment 20 and are configured to extend around the patient's sides. The two bra attachment portions 130 comprise a closure mechanism 132, such as a zipper, configured to connect the bra attachment portions 130 at the front of the patient. Alternatively, the closure mechanism 132 may comprise one or more clasps as described herein. It is to be appreciated that the closure mechanism 132 may be of any type known to be suitable to those having ordinary skill in the art.

The cups 131 of the bra attachment portions 130 incorporate suitable structure for supporting the patient's breasts in a comfortable and stylized manner that is typical of various bra garments known in the art. Accordingly, the cups 131 may include layers of foam padding, underwire, and/or plastic inserts typically used in bra garments to provide comfortable support for the patient's breasts and a suitable fit for the patient. The cups 131 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portions 130 may be worn under the patient's normal clothing in a manner that minimizes noticeable protrusion of the bra attachment portions 130 and interference with the fit and comfort of the patient's clothing. In other words, the bra attachment portion 130 may be structured to fit closely to the patient's body and to conform to the shape of the patient's front upper torsal anatomy such that the outward extension of the bra attachment portion 130 from the patient's body is minimized so that the bra attachment portion 130 does not extend or only minimally extends into a space generally occupied by the patient's normal clothing.

The bra attachment portions 130 may be formed from any material or materials known to be suitable to those having ordinary skill in the art. In particular, the bra attachment portions 130 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portions 130 may be provided in multiple sizes and with multiple cup sizes to fit patients of different body types. For instance, the bra attachment portions 130 may be provided in multiple sizes S, M, L, XL, XXL, etc. corresponding to the size of the support garment 20. The bra attachment portions 130 may also be provided having different sized cups A, B, C, D, etc. typically found in bra garments. The bra attachment portions 130 may also each include an adjustment mechanism, such as the inflatable insert 200 shown in FIGS. 22A and 22B or the plurality of drawstrings 212 shown in FIG. 23, configured to adjust a fit of the bra attachment portions 130 to the patient.

The support garment incorporating the bra attachment portions 130 illustrated in FIG. 14 is advantageous because it structurally incorporates the bra attachment portions into the existing support garment 20 and because it will be easier to fasten for patients having arthritis or similar conditions affecting their ability to manipulate the bra attachment portions 130.

### Support Garment Incorporating Bra Attachment Portions Connected Between the Belt and Straps:

FIGS. 15 and 16 illustrate a support garment for supporting a patient wearable defibrillator according to an example of the present disclosure. The illustrated support garment is a combination of the support garment 20 discussed above with reference to FIGS. 2-5 and two bra attachment portions 140 each comprising a cup 141 configured to contact and support one of the patient's breasts. As discussed above, the support garment 20 is made from a fabric having an outside surface and an inside surface and is configured to be worn about a chest of a patient P. The support garment 20 comprises a back portion 21, a belt 22 defined by side portions extending from the back portion 21 around a front of the patient P, and shoulder straps 23 attached to the back portion 21 and the belt 22. The back portion 21 and the belt 22 are configured to support a plurality of sensing electrodes 12, shown in FIGS. 1 and 2, and at least one therapeutic defibrillator electrode 11, also shown in FIGS. 1 and 2.

The two bra attachment portions 140 are built or integrated into the front of the support garment 20 to form a hybrid support garment with the bra attachment portions 140 being disposed between the straps 23 and the belt 22. Each bra attachment portion 140 is connected to a respective portion of the belt 22 at a bottom side thereof. As shown, the bra attachment portions 140 are connected to the respective portions of the belt 22 by seams of stitching 143. The bra attachment portions 140 are integrated into the respective portions of the belt 22 such that the bra attachment portions 140 and the belt 22 may be provided with a common closure mechanism 142, such as a zipper, configured to connect the bra attachment portions and the ends of the belt 22 at the front of the patient P. Alternatively, the closure mechanism 142 may comprise one or more clasps as described herein. It is to be appreciated that the closure mechanism 142 may be of any type known to be suitable to those having ordinary skill in the art.

Shortened straps 23 are provided to connect the back portion 21 of the support garment 20 to a top side of a respective bra attachment portion 140. The bra attachment portions 140 may be connected to the respective straps 23 by stitching or by a suitable detachable fastening mechanism such as a mateable buckle 172 as shown in FIG. 19, mateable plastic slide connectors as shown in FIGS. 24A and 24B, one or more clasps as shown in FIGS. 25A and 25B, one or more buttons as shown in FIGS. 26A and 26B, one or more snaps as shown in FIGS. 27A and 27B, or another fastening mechanism known to be suitable to those having ordinary skill in the art.

The cups 141 of the bra attachment portions 140 incorporate suitable structure for supporting the patient's breasts in a comfortable and stylized manner that is typical of various bra garments known in the art. Accordingly, the cups 141 may include layers of foam padding, underwire, and/or plastic inserts typically used in bra garments to provide comfortable support for the patient's breasts and a suitable fit for the patient. The cups 141 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portions 140 may be worn under the patient's normal clothing in a manner that minimizes noticeable protrusion of the bra attachment portions 140 and interference with the fit and comfort of the patient's clothing. In other words, the bra attachment portion 140 may be structured to fit closely to the patient's body and to conform to the shape of the patient's front upper torsal anatomy such that the outward extension of the bra attachment portion 140 from the patient's body is minimized so that the bra attachment portion 140 does not extend or only minimally extends into a space generally occupied by the patient's normal clothing.

The bra attachment portions 140 may be formed from any material or materials known to be suitable to those having ordinary skill in the art. In particular, the bra attachment portions 140 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portions 140 may be provided in multiple sizes and with multiple cup sizes to fit patients of different body types. For instance, the bra attachment portions 140 may be provided in multiple sizes S, M, L, XL, XXL, etc. corresponding to the size of the support garment 20. The bra attachment portions 140 may also be provided having different sized cups A, B, C, D, etc. typically found in bra garments. The bra attachment portions 140 may also each include an adjustment mechanism, such as the inflatable insert 200 shown in FIGS. 22A and 22B or the plurality of drawstrings 212 shown in FIG. 23, configured to adjust a fit of the bra attachment portions 140 to the patient P.

The support garment incorporating the bra attachment portions 140 illustrated in FIGS. 15 and 16 is advantageous because it more fully integrates the bra attachment portions 140 into the support garment 20 and because it will be easier to fasten for patients having arthritis or similar conditions affecting their ability to manipulate the garment.

### Support Garment Incorporating a Halter Style Bra:

FIG. 17 illustrates a support garment for supporting a patient wearable defibrillator according to an example of the present disclosure. The illustrated support garment is a combination of the support garment 20 discussed above with reference to FIGS. 2-5 and a bra attachment portion 150 in the form of a halter style bra.

The bra attachment portion 150 is configured to support front upper torsal female anatomy and extends between the straps 23 and the belt 22 of the support garment 20 at the front of the patient. The bra attachment portion 150 includes two cups 151 that are configured to contact and support the patient's breasts. The cups 151 incorporate suitable structure for supporting the patient's breasts in a comfortable and stylized manner that is typical of various bra garments known in the art. Accordingly, the cups 151 may include layers of foam padding, underwire, and/or plastic inserts typically used in bra garments to provide comfortable support for the patient's breasts and a suitable fit for the patient. The cups 151 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portion 150 may be worn under the patient's normal clothing in a manner that minimizes noticeable protrusion of the bra attachment portion 150 and interference with the fit and comfort of the patient's clothing. In other words, the bra attachment portion 150 may be structured to fit closely to the patient's body and to conform to the shape of the patient's front upper torsal anatomy such that the outward extension of the bra attachment portion 150 from the patient's body is minimized so that the bra attachment portion 150 does not extend or only minimally extends into a space generally occupied by the patient's normal clothing. Gel strips (not shown) may be provided on the sides of the bra attachment portion 150 for fit and to hold the bra attachment portion 150 against the patient's skin.

The bra attachment portion 150 includes a single halter strap 152 connected to the top of the bra attachment portion 150 proximate to both lateral sides thereof. The halter strap 152 is configured to extend around a neck of the patient.

The bra attachment portion 150 also includes one or more fastening mechanisms 153 disposed along the bottom thereof for connecting the bra attachment portion 150 to the belt 22. The belt 22 includes corresponding fastening mechanisms 154. As discussed above with respect to FIGS. 6 and 7, the fastening mechanisms 153, 154 may comprise strips of hook and pile fasteners, plastic male and female mateable slide connectors 215, 216 of the type shown in FIGS. 24A and 24B, a plurality of clasps of the type shown in FIGS. 25A and 25B, a plurality of buttons of the type shown in FIGS. 26A and 26B, and/or a plurality of snaps of the type shown in FIGS. 27A and 27B. The bra attachment portion 150 may also be connected to the belt 22 by press fit connectors or by mateable buckles.

The bra attachment portion 150 may be formed from any material or materials known to be suitable to those having ordinary skill in the art. In particular, the bra attachment portion 150 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portion 150 may be provided in multiple sizes and with multiple cup sizes to fit patients of different body types. For instance, the bra attachment portion 150 may be provided in multiple sizes S, M, L, XL, XXL, etc. corresponding to the size of the support garment 20. The bra attachment portion 150 may also be provided having different sized cups A, B, C, D, etc. typically found in bra garments. The bra attachment portion 150 may also include an adjustment mechanism, such as the inflatable insert 200 shown in FIGS. 22A and 22B or the plurality of drawstrings 212 shown in FIG. 23, configured to adjust a fit of the bra attachment portion 150 to the patient P.

The bra attachment portion 150 illustrated in FIG. 17 is advantageous because it requires minimal modification to the support garment 20 so that different sized support garments 20 may be matched with different sized bra attachment portions 150 at the time of fitting and utilizes the support garment 20 to supplement the structure of the bra attachment portion 150. The halter strap 152 provides support without interfering with the straps 23 of the support garment 20.

### Support Garment Incorporating Bra Attachment Portions Connected to the Belt and Straps:

FIG. 18 illustrates a support garment for supporting a patient wearable defibrillator according to an example of the present disclosure. The illustrated support garment is a combination of the support garment 20 discussed above with reference to FIGS. 2-5 and two bra attachment portions 160 each comprising a cup 161 configured to contact and support one of the patient's breasts.

Each bra attachment portion 160 is connected to a respective portion of the belt 22 at a bottom side thereof and to a respective strap 23 at a lateral side thereof. The bra attachment portions 160 may be permanently connected during fitting to the respective portions of the belt 22 and to the respective straps 23 by one or more press fit connectors 163, as shown in FIG. 18, or by an alternative connection such as steams of stitching as shown in FIGS. 15 and 16. Alternatively, the bra attachment portions 160 may be detachably connected to the respective portions of the belt 22 and/or the respective straps 23 by a suitable detachable fastening mechanism such as hook and pile fasteners, one or more mateable buckles 172 as shown in FIG. 19, mateable plastic slide connectors as shown in FIGS. 24A and 24B, one or more clasps as shown in FIGS. 25A and 25B, one or more buttons as shown in FIGS. 26A and 26B, one or more snaps as shown in FIGS. 27A and 27B, or other fastening mechanism knowns to be suitable to those having ordinary skill in the art.

The two bra attachment portions 160 comprise a closure mechanism 162, such as a zipper, configured to connect the bra attachment portions 160 at the front of the patient. Alternatively, the closure mechanism 162 may comprise one or more clasps as described herein. It is to be appreciated that the closure mechanism 162 may be of any type known to be suitable to those having ordinary skill in the art.

The cups 161 of the bra attachment portions 160 incorporate suitable structure for supporting the patient's breasts in a comfortable and stylized manner that is typical of various bra garments known in the art. Accordingly, the cups 161 may include layers of foam padding, underwire, and/or plastic inserts typically used in bra garments to provide comfortable support for the patient's breasts and a suitable fit for the patient. The cups 161 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portions 160 may be worn under the patient's normal clothing in a manner that minimizes noticeable protrusion of the bra attachment portions 160 and interference with the fit and comfort of the patient's clothing. In other words, the bra attachment portion 160 may be structured to fit closely to the patient's body and to conform to the shape of the patient's front upper torsal anatomy such that the outward extension of the bra attachment portion 160 from the patient's body is minimized so that the bra attachment portion 160 does not extend or only minimally extends into a space generally occupied by the patient's normal clothing.

The bra attachment portions 160 may be formed from any material or materials known to be suitable to those having ordinary skill in the art. In particular, the bra attachment portions 160 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portions 160 may be provided in multiple sizes and with multiple cup sizes to fit patients of different body types. For instance, the bra attachment portions 160 may be provided in multiple sizes S, M, L, XL, XXL, etc. corresponding to the size of the support garment 20. The bra attachment portions 160 may also be provided having different sized cups A, B, C, D, etc. typically found in bra garments. The bra attachment portions 160 may also each include an adjustment mechanism, such as the inflatable insert 200 shown in FIGS. 22A and 22B or the plurality of drawstrings 212 shown in FIG. 23, configured to adjust a fit of the bra attachment portions 160 to the patient.

The support garment incorporating the bra attachment portions 160 illustrated in FIG. 18 is advantageous because it requires minimal modification to the existing support garment 20 so that different sized support garments 20 may be matched with different sized bra attachment portions 160 at the time of fitting and utilizes support garment 20 to supplement the structure of the bra attachment portions 160. When press fit connectors 163 are utilized to permanently connect the bra attachment portions 160 to the belt 22 and the straps 23, the bra attachment portions 160 become a permanent part of the support garment 20 and do not need to be re-fastened or adjusted.

### Support Garment Incorporating Bra Attachment Portions Detachably Connected to the Belt and Straps:

FIG. 19 illustrates a support garment for supporting a patient wearable defibrillator according to an example of the present disclosure. The illustrated support garment is a combination of the support garment 20 discussed above with reference to FIGS. 2-5 and two bra attachment portions 170 each comprising a cup 171 configured to contact and support one of the patient's breasts. As discussed above, the support garment 20 is made from a fabric having an outside surface and an inside surface and is configured to be worn about a chest of a patient P. The support garment 20 comprises a back portion 21, a belt 22 defined by side portions extending from the back portion 21 around a front of the patient P, and shoulder straps 23 attached to the back portion 21 and the belt 22. The back portion 21 and the belt 22 are configured to support a plurality of sensing electrodes 12, shown in FIGS. 1 and 2, and at least one therapeutic defibrillator electrode 11, also shown in FIGS. 1 and 2.

The two bra attachment portions 170 are detachably connected to the front of the support garment 20 between the straps 23 and the belt 22. Each bra attachment portion 170 is connected to a respective portion of the belt 22 at a bottom side thereof by quick disconnect features, such as one or more mateable buckles 172 attached or adhered to the bra attachment portions 170 and the portions of the belt 22. Each buckle 172 includes a male portion having a protruding button or tab and a female portion that includes a recess or aperture therein for receiving the button or tab on the male portion when the male portion is inserted into the female portion. The bra attachment portions 170 may be connected to the belt 22 by a different suitable detachable fastening mechanism such as hook and pile fasteners, mateable plastic slide connectors as shown in FIGS. 24A and 24B, one or more clasps as shown in FIGS. 25A and 25B, one or more buttons as shown in FIGS. 26A and 26B, one or more snaps as shown in FIGS. 27A and 27B, or other fastening mechanisms known to be suitable to those having ordinary skill in the art.

Shortened straps 23 are provided to connect the back portion 21 of the support garment 20 to a top side of a respective bra attachment portion 170. The bra attachment portions 170 are also connected to the respective straps 23 by mateable buckles 172. Alternatively, the bra attachment portions 170 may be detachably connected to the respective straps 23 by mateable plastic slide connectors as shown in FIGS. 24A and 24B, one or more clasps as shown in FIGS. 25A and 25B, one or more buttons as shown in FIGS. 26A and 26B, one or more snaps as shown in FIGS. 27A and 27B, or another fastening mechanism known to be suitable to those having ordinary skill in the art. A halter style strap of the type described above with reference to FIG. 17 may be provided for additional support.

The two bra attachment portions 170 comprise a closure mechanism 173, such as a zipper, configured to connect the bra attachment portions 170 at the front of the patient. Alternatively, the closure mechanism 173 may comprise one or more clasps as described herein. It is to be appreciated that the closure mechanism 173 may be of any type known to be suitable to those having ordinary skill in the art.

The cups 171 of the bra attachment portions 170 incorporate suitable structure for supporting the patient's breasts in a comfortable and stylized manner that is typical of various bra garments known in the art. Accordingly, the cups 171 may include layers of foam padding, underwire, and/or plastic inserts typically used in bra garments to provide comfortable support for the patient's breasts and a suitable fit for the patient. The cups 171 may be structured, through the use of the above-mentioned layers of foam padding, underwire, and/or plastic inserts, in a smooth rounded shape to match the contours of the patient's front upper torsal anatomy and to define a smoothed, stylized silhouette such that the bra attachment portions 170 may be worn under the patient's normal clothing in a manner that minimizes noticeable protrusion of the bra attachment portions 170 and interference with the fit and comfort of the patient's clothing. In other words, the bra attachment portion 170 may be structured to fit closely to the patient's body and to conform to the shape of the patient's front upper torsal anatomy such that the outward extension of the bra attachment portion 170 from the patient's body is minimized so that the bra attachment portion 170 does not extend or only minimally extends into a space generally occupied by the patient's normal clothing.

The bra attachment portions 170 may be formed from any material or materials known to be suitable to those having ordinary skill in the art. In particular, the bra attachment portions 170 may be formed from the same elastic, low spring rate material as the support garment 20, discussed above with reference to FIGS. 2-5, such as a blend of spandex material with nylon and/or polyester materials. As also discussed above, the stitching may be made from a cotton wrapped polyester core thread. The bra attachment portions 170 may be provided in multiple sizes and with multiple cup sizes to fit patients of different body types. For instance, the bra attachment portions 170 may be provided in multiple sizes S, M, L, XL, XXL, etc. corresponding to the size of the support garment 20. The bra attachment portions 170 may also be provided having different sized cups A, B, C, D, etc. typically found in bra garments. The bra attachment portions 170 may also each include an adjustment mechanism, such as the inflatable insert 200 shown in FIGS. 22A and 22B or the plurality of drawstrings 212 shown in FIG. 23, configured to adjust a fit of the bra attachment portions 170 to the patient P.

The support garment incorporating the bra attachment portions 170 illustrated in FIG. 19 is advantageous because it structurally incorporates the bra attachment portions 170 into the support garment 20 and because it will be easier to fasten/connect for patients having arthritis or similar conditions affecting their ability to manipulate the garment.

### Vest Garment Incorporating Support Structure for a Patient Wearable Defibrillator

FIG. 20 illustrates a support garment 180 for a patient wearable defibrillator in the form of a vest according to an example of the present disclosure. The defibrillator includes an electrode assembly 10 having a plurality of sensing electrodes 12 configured to monitor a cardiac function of the patient P and at least one therapeutic defibrillator electrode 11, as discussed above with reference to FIGS. 1 and 2. The vest 180 is made from a fabric having an outside surface and an inside surface and is configured to be worn about a chest of the patient. As discussed above with reference to the support garment 101 illustrated in FIGS. 8-11, the vest 180 is configured to support and hold the sensing electrodes 12 and the at least one therapeutic defibrillator 11 against the patient's skin at the proper locations on the patient's body in a manner similar to the support garment 20 discussed above with reference to FIGS. 2-5.

As shown, vest 180 includes a back portion 181 and a front portion 182 connected to the back portion 181. The front portion 182 of the vest 180 is configured to support front upper torsal female anatomy. The inside surface of the vest 180 includes at least one support member on the inside surface thereof for receiving and supporting at least one therapeutic defibrillator electrode 11 on the vest 180 and one or more attachment points for attaching the sensing electrodes 12 to the inside surface of the vest 180 and distributing the sensing electrodes 12 around the circumference of the patient's chest. According to one example, the inside surface of the vest 180 incorporates the same pockets 104, 105, attachment points 108, and flap 112 in the same positions as the above-described support garment 101 illustrated in FIGS. 8-11.

The vest 180 includes two cups 183 that are configured to contact and support the patient's breasts. The cups 183 incorporate suitable structure for supporting the patient's breasts in a comfortable and stylized manner that is typical of various bra garments known in the art. Accordingly, the cups 183 may include layers of foam padding, underwire, and/or plastic inserts typically used in bra garments to provide comfortable support for the patient's breasts and a suitable fit for the patient. The cups 183 may be structured, through the use of the above-mentioned layers of foam padding, underwire, and/or plastic inserts, in a smooth rounded shape to match the contours of the patient's front upper torsal anatomy and to define a smoothed, stylized silhouette such that the vest 180 may be worn under the patient's normal clothing in a manner that minimizes noticeable protrusion of the vest 180 and interference with the fit and comfort of the patient's clothing. In other words, the vest 180 may be structured to fit closely to the patient's body and to conform to the shape of the patient's front upper torsal anatomy such that the outward extension of the vest 180 from the patient's body is minimized so that the vest 180 does not extend or only minimally extends into a space generally occupied by the patient's normal clothing.

As shown, the vest 180 is split in the front and provided with a front closure mechanism 184, such as a zipper, a plurality of clasps or a similar fastener, to connect the separate halves of the front portion 182 at the front of the patient in order to facilitate wearing and removing of the vest 180. An elastic drawstring 185 may also be provided around the bottom of the vest 180 to allow the patient to more closely draw the vest 180 to the patient's body. The drawstring 185 may be provided with clamps or clips to hold the drawstring at the desired length.

The vest 180 may be formed from any material or materials known to be suitable to those having ordinary skill in the art. In particular, the portions of the vest 180 which support the electrode assembly 10 on the vest 180 and which define the cups 183 may be formed from the same elastic, low spring rate material as the support garment 20 discussed above with reference to FIGS. 2-5, such as a blend of spandex material with nylon and/or polyester materials. As also discussed above, the stitching may be made from a cotton wrapped polyester core thread. Other portions of the vest 180, such as the mesh portions 186 around the patient's shoulders and underarms may be formed from a lighter, more breathable mesh material to reduce the weight of the vest 180 and to allow the vest 180 to be more breathable and cooler while still helping to hold the position of the vest 180 on the patient's body.

The vest 180 may be provided in multiple sizes and with multiple cup sizes to fit patients of different body types. For instance, the vest 180 may be provided in multiple sizes S, M, L, XL, XXL, etc. The vest 180 may also be provided having different cup sizes A, B, C, D, etc. typically found in bra garments. Each cup 183 of the vest 180 may also include an adjustment mechanism configured to adjust a fit of the cup 183 to the patient. The adjustment mechanism may comprise an inflatable insert 200 as discussed above with reference to FIGS. 22A and 22B or a plurality of drawstrings 212 as discussed above with reference to FIG. 23.

The vest 180 is advantageous because it fully integrates the support structures for the electrode assembly 10 found in the support garment 20 with a garment that also functions to support front upper torsal female anatomy. The vest 180 also serves to more securely support and position the electrode assembly 10 on the patient's body and will be easier to put on and fasten/connect for patients having arthritis or similar conditions affecting their ability to manipulate the garment.

### Multi-Piece Support Garment:

FIG. 21 illustrates a support garment 190 for a patient wearable defibrillator in the form of a modular, multi-piece garment customizable garment. The defibrillator includes an electrode assembly 10 having a plurality of sensing electrodes 12 configured to monitor a cardiac function of the patient P and at least one therapeutic defibrillator electrode 11, as discussed above with reference to FIGS. 1 and 2. The support garment 190 is made from multiple pieces fabric that are assembled during fitting to form a support garment that is customized to the size and body of a particular patient, male or female. The support garment 190, when assembled, has an outside surface and an inside surface and is configured to be worn about a chest of the patient. As discussed above with reference to the support garment 101 illustrated in FIGS. 8-11 and the vest 180 illustrated in FIG. 20, the support garment 190 is configured to support and hold the sensing electrodes 12 and the at least one therapeutic defibrillator 11 against the patient's skin at the proper locations on the patient's body in a manner similar to the support garment 20 discussed above with reference to FIGS. 2-5.

As shown, the support garment 190 includes a standard back portion 191 for male and female patients, a front/side belt 194 for male patients or a front/side tank or halter 192 incorporating cups 193 for female patients, and optional straps 195 for connecting the back portion 191 to the front/side belt 194 or front/side tank or halter 192. The front/side tank or halter 192 for female patients may incorporate straps for connecting to the back portion 191. The straps utilized in the support garment 190 may have an adjustable size. All of the above-mentioned components 191 come in different sizes to accommodate patients of different sizes and body types. The front/side tank or halter 192 for female patients is configured to support front upper torsal female anatomy.

The inside surface of the support garment 190 includes at least one support member on the inside surface thereof for receiving and supporting at least one therapeutic defibrillator electrode on the support garment 190 and one or more attachment points for attaching the sensing electrodes 12 to the inside surface of the support garment 190 and distributing the sensing electrodes 12 around the circumference of the patient's chest. According to one example, the back portion 191, the front/side belt 194, and the front/side tank or halter 192 are formed to incorporate the same the same pockets 104, 105, attachment points 108, and flap 112 in the same positions as the above-described support garment 101 illustrated in FIGS. 8-11.

The front/side tank or halter 192 includes two cups 193 that are configured to contact and support the patient's breasts. The cups 193 incorporate suitable structure for supporting the patient's breasts in a comfortable and stylized manner that is typical of various bra garments known in the art. Accordingly, the cups 193 may include layers of foam padding, underwire, and/or plastic inserts typically used in bra garments to provide comfortable support for the patient's breasts and a suitable fit for the patient. The cups 193 may be structured, through the use of the above-mentioned layers of foam padding, underwire, and/or plastic inserts, in a smooth rounded shape to match the contours of the patient's front upper torsal anatomy and to define a smoothed, stylized silhouette such that the support garment 190 may be worn under the patient's normal clothing in a manner that minimizes noticeable protrusion of the support garment 190 and interference with the fit and comfort of the patient's clothing. In other words, the support garment 190 may be structured to fit closely to the patient's body and to conform to the shape of the patient's front upper torsal anatomy such that the outward extension of the support garment 190 from the patient's body is minimized so that the support garment 190 does not extend or only minimally extends into a space generally occupied by the patient's normal clothing. The front/side tank or halter 192 may be provided having different cup sizes A, B, C, D, etc. typically found in bra garments. Each cup 193 of the front/side tank or halter may also include an adjustment mechanism configured to adjust a fit of the cup 193 to the patient. The adjustment mechanism may comprise an inflatable insert 200 as discussed above with reference to FIGS. 22A and 22B or a plurality of drawstrings 212 as discussed above with reference to FIG. 23.

As shown, the front/side belt 194 and the front/side tank or halter 192 may be provided as two separate pieces split in the front and provided with a front closure mechanism 196, such as a zipper, a plurality of clasps or a similar fastener, to connect the separate halves of the front/side belt 194 or the front/side tank or halter 192 at the front of the patient in order to facilitate wearing and removing of the support garment 190.

The components 191, 192, 194, 195 of the support garment 190 may be formed from any material or materials known to be suitable to those having ordinary skill in the art. In particular, the components 191, 192, 194, 195 may be formed from the same elastic, low spring rate material as the support garment 20 discussed above with reference to FIGS. 2-5, such as a blend of spandex material with nylon and/or polyester materials. As also discussed above, the stitching may be made from a cotton wrapped polyester core thread.

During fitting of the support garment 190, the standard components 191, 192, 194, 195 of different types and sizes are selected for the patient and are then assembled, such as by sewing or stitching, to form a custom garment.

The support garment 190 is advantageous because it provide for a customized garment for all patients made from standardized components and allows for the breast support structure to be fully integrated with the components for attaching the electrode assembly 10 to the garment 190. The support garment 190 will also be easier to put on and fasten/connect for patients having arthritis or similar conditions affecting their ability to manipulate the garment.

Although a wearable medical device and a support garment for such a device have been described in detail for the purpose of illustration based on what is currently considered to be the most practical examples, it is to be understood that such detail is solely for that purpose and that the subject matter of this disclosure is not limited to the disclosed examples, but, on the contrary, is intended to cover modifications and arrangements, as defined by the appended claims.

## Claims

1. A support garment (20) for supporting a patient wearable defibrillator, the support garment being made from a fabric having an outside surface and an inside surface and being configured to be worn about a chest of a patient (P), the support garment comprising:
a back portion (21);
a belt (22) defined by side portions extending from the back portion around a front of the patient; and
shoulder straps (23) configured to be attached to the back portion and the belt,
wherein the back portion and the belt are configured to support a plurality of sensing electrodes (12) and at least one therapeutic defibrillator electrode (11) on the inside surface thereof,
wherein the support garment further comprises at least one bra portion (50) detachably connected to the belt and the shoulder straps to form a single structure of the bra portion (50) and the support garment (20) for wearing by the patient and wherein the at least one bra portion (50) is detachable from the support garment, and
wherein the at least one bra portion is configured to support front upper torsal female anatomy, the at least one bra portion comprising two cups (51) configured to support the patient's breasts.

2. The support garment (20) according to claim 1, wherein the shoulder straps (23) are adjustable.

3. The support garment (20) according to claim 1 or 2, wherein the shoulder straps (23) are configured to be selectively attached to the belt (22) at the front of the patient.

4. The support garment (20) according to any one of claims 1-3, wherein the fabric of the support garment comprises an elastic, low spring rate material.

5. The support garment (20) according to any one of claims 1-4, further comprising at least one pocket configured to receive the at least one therapeutic defibrillator electrode at the front or back of the patient.

6. The support garment (20) according to any one of claims 1-5, wherein the back portion (21) and belt (22) of the support garment are configured to distribute the plurality of sensing electrodes (12) around a circumference of the chest of the patient.

7. The support garment (20) according to any one of claims 1-6, wherein the at least one bra portion is configured to be detachably connected to the belt (22) at the front of the patient.

8. The support garment (20) according to claim 7, wherein the at least one bra portion is detachably connected to the belt (22) by at least one of the following: hook and pile fasteners, a clasp, a button, a snap, a mateable buckle, and a mateable slide connector.

9. The support garment (20) according to claim 7 or 8, wherein the at least one bra portion further comprises a halter strap connected to a top of the at least one bra portion proximate to both lateral sides thereof, the halter strap being configured to extend around a neck of the patient.

10. The support garment (20) according to claim 1, wherein the at least one bra portion comprises flaps at both lateral sides thereof, the flaps being configured to wrap around the shoulder straps (23) and comprising fastening mechanisms to secure ends of the flaps.

11. The support garment (20) according to claim 1, wherein the at least one bra portion, the belt (22), and the shoulder straps (23) comprise corresponding mateable slide connectors configured to connect the at least one bra portion to the belt and the shoulder straps.

12. The support garment (20) according to claim 1, the at least one bra portion comprises an adjustment mechanism configured to adjust a fit of the bra portion to the patient.

13. The support garment (20) according to any one of claims 1-6, wherein the at least one bra portion comprises two bra portions configured to be detachably connected to the shoulder straps and the belt (22), each of the two bra portions comprising a respective one of the two cups.

14. The support garment (20) according to claim 13, wherein the two bra portions comprise a closure mechanism configured to detachably connect the two bra portions at the front of the patient.

15. The support garment (20) according to claim 14, wherein the belt (22) and the two bra portions comprise a common closure mechanism configured to connect the two bra portions and ends of the belt (22) at the front of the patient.

16. The support garment (20) according to any one of claims 14-15, wherein each of the two bra portions is configured to be detachably connected to a respective belt portion by at least one of the following: hook and pile fasteners, a clasp, a button, a snap, a mateable buckle, and a mateable slide connector.

## Patentansprüche

1. Stützkleidung (20) zum Stützen eines von einem Patienten tragbaren Defibrillators, wobei die Stützkleidung aus einem Gewebe mit einer äußeren Oberfläche und einer inneren Oberfläche hergestellt ist und so konfiguriert ist, dass sie um die Brust eines Patienten (P) getragen werden kann, die Stützkleidung umfassend:
einen hinteren Abschnitt (21);
einen Band (22), das durch Seitenabschnitte definiert ist, die sich von dem hinteren Abschnitt um eine Vorderseite des Patienten herum erstrecken; und
Schulterriemen (23), die so konfiguriert sind, dass sie am hinteren Abschnitt und dem Band befestigt werden können,
wobei der hintere Abschnitt und das Band so konfiguriert sind, dass sie eine Vielzahl von Sensorelektroden (12) und mindestens eine therapeutische Defibrillatorelektrode (11) auf ihrer inneren Oberfläche stützen,
wobei die Stützkleidung ferner mindestens einen BH-Abschnitt (50) umfasst, der abnehmbar mit dem Band und den Schulterriemen verbunden ist, um eine einzige Struktur aus dem BH-Abschnitt (50) und der Stützkleidung (20) zum Tragen durch den Patienten zu bilden, und wobei der mindestens eine BH-Abschnitt (50) von der Stützkleidung abnehmbar ist, und
wobei der mindestens eine BH-Abschnitt so konfiguriert ist, dass er die vordere obere torsale weibliche Anatomie stützt, wobei der mindestens eine BH-Abschnitt zwei Körbchen (51) umfasst, die so konfiguriert sind, dass sie die Brüste der Patientin stützen.

2. Stützkleidung (20) nach Anspruch 1, wobei die Schulterriemen (23) verstellbar sind.

3. Stützkleidung (20) nach Anspruch 1 oder 2, wobei die Schulterriemen (23) so konfiguriert sind, dass sie selektiv an dem Band (22) an der Vorderseite des Patienten befestigt werden können.

4. Stützkleidung (20) nach einem der Ansprüche 1 bis 3, wobei das Gewebe der Stützkleidung ein elastisches Material mit niedriger Federrate umfasst.

5. Stützkleidung (20) nach einem der Ansprüche 1 bis 4, ferner umfassend mindestens eine Tasche, die so konfiguriert ist, dass sie die mindestens eine therapeutische Defibrillatorelektrode an der Vorder- oder Rückseite des Patienten empfängt.

6. Stützkleidung (20) nach einem der Ansprüche 1 bis 5, wobei der hintere Abschnitt (21) und der Band (22) der Stützkleidung so konfiguriert sind, dass die Vielzahl von Sensorelektroden (12) um den Umfang der Brust des Patienten herum verteilt sind.

7. Stützkleidung (20) nach einem der Ansprüche 1 bis 6, wobei der mindestens eine BH-Abschnitt so konfiguriert ist, dass er abnehmbar mit dem Band (22) an der Vorderseite des Patienten verbunden ist.

8. Stützkleidung (20) nach Anspruch 7, wobei der mindestens eine BH-Abschnitt durch mindestens eines von Folgendem lösbar mit dem Band (22) verbunden ist: Haken- und Stapelverschlüsse, eine Schließe, ein Knopf, ein Schnappverschluss, eine steckbare Schnalle und ein steckbarer Schiebeverbinder.

9. Stützkleidung (20) nach Anspruch 7 oder 8, wobei der mindestens eine BH-Abschnitt ferner einen Halteriemen umfasst, der mit der Oberseite des mindestens einen BH-Abschnitts in der Nähe seiner beiden Seitenabschnitte verbunden ist, wobei der Halteriemen so konfiguriert ist, dass er sich um den Hals des Patienten erstreckt.

10. Stützkleidung (20) nach Anspruch 1, wobei der mindestens eine BH-Abschnitt Klappen an seinen beiden Seitenabschnitten umfasst, wobei die Klappen so konfiguriert sind, dass sie sich um die Schulterriemen (23) wickeln und Befestigungsmechanismen umfassen, um die Enden der Klappen zu sichern.

11. Stützkleidung (20) nach Anspruch 1, wobei der mindestens eine BH-Abschnitt, der Band (22) und die Schulterriemen (23) entsprechende, zusammenpassende Schiebeverbinder umfassen, die konfiguriert sind, um den mindestens einen BH-Abschnitt mit dem Band und den Schulterriemen zu verbinden.

12. Stützkleidung (20) nach Anspruch 1, wobei der mindestens eine BH-Abschnitt einen Einstellmechanismus umfasst, der so konfiguriert ist, dass er die Passform des BH-Abschnitts an den Patienten anpasst.

13. Stützkleidung (20) nach einem der Ansprüche 1 bis 6, wobei der mindestens eine BH-Abschnitt zwei BH-Abschnitte umfasst, die so konfiguriert sind, dass sie abnehmbar mit den Schulterriemen und dem Band (22) verbunden sind, wobei jeder der beiden BH-Abschnitte jeweils eines der beiden Körbchen umfasst.

14. Stützkleidung (20) nach Anspruch 13, wobei die beiden BH-Abschnitte einen Verschlussmechanismus umfassen, der so konfiguriert ist, dass die beiden BH-Abschnitte an der Vorderseite des Patienten lösbar miteinander verbunden sind.

15. Stützkleidung (20) nach Anspruch 14, wobei das Band (22) und die beiden BH-Abschnitte einen gemeinsamen Verschlussmechanismus umfassen, der so konfiguriert ist, dass die beiden BH-Abschnitte und die Enden des Bandes (22) an der Vorderseite des Patienten verbunden sind.

16. Stützkleidung (20) nach einem der Ansprüche 14 bis 15, wobei jeder der beiden BH-Abschnitte so konfiguriert ist, dass er mit einem entsprechenden Bandabschnitt durch mindestens eines von Folgendem lösbar verbunden ist: Haken- und Stapelverschlüsse, eine Schließe, ein Knopf, ein Schnappverschluss, eine steckbare Schnalle und ein steckbarer Schiebeverbinder.

## Revendications

1. Vêtement de soutien (20) destiné à soutenir un défibrillateur portable d'un patient, le vêtement de soutien étant constitué d'un tissu ayant une surface extérieure et une surface intérieure et étant configuré pour être porté autour de la poitrine d'un patient (P), le vêtement de soutien comprenant :
une partie arrière (21) ;
une ceinture (22) définie par des parties latérales s'étendant depuis la partie arrière autour d'un devant de la patiente ; et
des bretelles (23) configurées pour être attachées à la partie arrière et à la ceinture,
dans lequel la partie arrière et la ceinture sont configurées pour supporter une pluralité d'électrodes de détection (12) et au moins une électrode de défibrillateur thérapeutique (11) sur leur surface intérieure,
dans lequel le vêtement de soutien comprend en outre au moins une partie soutien-gorge (50) reliée de manière détachable à la ceinture et aux bretelles pour former une structure unique de la partie soutien-gorge (50) et du vêtement de soutien (20) destinée à être portée par la patiente et dans lequel l'au moins une partie soutien-gorge (50) est détachable du vêtement de soutien, et
dans lequel l'au moins une partie soutien-gorge est configurée pour soutenir l'anatomie féminine du torse supérieure avant, l'au moins une partie soutien-gorge comprenant deux bonnets (51) configurés pour soutenir les seins de la patiente.

2. Vêtement de soutien (20) selon la revendication 1, dans lequel les bretelles (23) sont réglables.

3. Vêtement de soutien (20) selon la revendication 1 ou 2, dans lequel les bretelles (23) sont configurées pour être fixées sélectivement à la ceinture (22) à l'avant de la patiente.

4. Vêtement de soutien (20) selon l'une quelconque des revendications 1 à 3, dans lequel le tissu du vêtement de soutien comprend un matériau élastique à faible taux de ressort.

5. Vêtement de soutien (20) selon l'une quelconque des revendications 1 à 4, comprenant en outre au moins une poche configurée pour recevoir l'au moins une électrode de défibrillateur thérapeutique à l'avant ou à l'arrière de la patiente.

6. Vêtement de soutien (20) selon l'une quelconque des revendications 1 à 5, dans lequel la partie arrière (21) et la ceinture (22) du vêtement de soutien sont configurées pour distribuer la pluralité d'électrodes de détection (12) autour d'une circonférence de la poitrine de la patiente.

7. Vêtement de soutien (20) selon l'une quelconque des revendications 1 à 6, dans lequel l'au moins une partie soutien-gorge est configurée pour être reliée de manière détachable à la ceinture (22) à l'avant de la patiente.

8. Vêtement de soutien (20) selon la revendication 7, dans lequel l'au moins une partie soutien-gorge est reliée de manière détachable à la ceinture (22) par au moins l'un des éléments suivants : des fixations à boucles et à crochets, un fermoir, un bouton, un boutonpression, une boucle pouvant être accouplée et un connecteur coulissant pouvant être accouplé.

9. Vêtement de soutien (20) selon la revendication 7 ou 8, dans lequel l'au moins une partie soutien-gorge comprend en outre une sangle licou reliée à un sommet de l'au moins une partie soutien-gorge à proximité des deux côtés latéraux de celle-ci, la sangle licou étant configurée pour s'étendre autour du cou de la patiente.

10. Vêtement de soutien (20) selon la revendication 1, dans lequel l'au moins une partie soutien-gorge comprend des rabats sur ses deux côtés latéraux, les rabats étant configurés pour s'enrouler autour des bretelles (23) et comprenant des mécanismes de fixation pour fixer les extrémités des rabats.

11. Vêtement de soutien (20) selon la revendication 1, dans lequel l'au moins une partie soutien-gorge, la ceinture (22) et les bretelles (23) comprennent des connecteurs coulissants pouvant être accouplés correspondants, configurés pour relier l'au moins une partie soutien-gorge à la ceinture et aux bretelles.

12. Vêtement de soutien (20) selon la revendication 1, l'au moins une partie soutien-gorge comprend un mécanisme de réglage configuré pour régler un ajustement de la partie soutien-gorge sur la patiente.

13. Vêtement de soutien (20) selon l'une quelconque des revendications 1 à 6, dans lequel l'au moins une partie soutien-gorge comprend deux parties soutien-gorge configurées pour être reliées de manière détachable aux bretelles et à la ceinture (22), chacune des deux parties soutien-gorge comprenant l'un respectif des deux bonnets.

14. Vêtement de soutien (20) selon la revendication 13, dans lequel les deux parties soutien-gorge comprennent un mécanisme de fermeture configuré pour relier de manière détachable les deux parties soutien-gorge à l'avant de la patiente.

15. Vêtement de soutien (20) selon la revendication 14, dans lequel la ceinture (22) et les deux parties soutien-gorge comprennent un mécanisme de fermeture commun configuré pour relier les deux parties soutien-gorge et les extrémités de la ceinture (22) à l'avant de la patiente.

16. Vêtement de soutien (20) selon l'une quelconque des revendications 14 à 15, dans lequel chacune des deux parties soutien-gorge est configurée pour être reliée de manière détachable à une partie de ceinture respective par au moins l'un des éléments suivants : des fixations à boucles et à crochets, un fermoir, un bouton, un boutonpression, une boucle pouvant être accouplée et un connecteur coulissant pouvant être accouplé.
